# EUROPEAN PATENT SPECIFICATION

(11) **EP 1 462 525 B1**
(45) Date of publication and mention of the grant of the patent: **06.10.2010**
(21) Application number: 02785973.5
(22) Date of filing: 28.11.2002
(51) Int. Cl.: C12N 15/11

(54) **siRNA EXPRESSION SYSTEM AND PROCESS FOR PRODUCING FUNCTIONAL GENE KNOCKDOWN CELL OR THE LIKE USING THE SAME**
siRNA-EXPRESSIONSSYSTEM UND VERFAHREN ZUR HERSTELLUNG EINER ZELLE O. Ä. MIT KNOCKDOWN EINES FUNKTIONELLEN GENS UNTER VERWENDUNG DESSELBEN
SYSTEME D'EXPRESSION D'ARNSI ET PROCEDE DE PRODUCTION DE CELLULE KNOCKDOWN A GENE FONCTIONNEL OU ANALOGUE UTILISANT CE SYSTEME

(30) Priority: 28.11.2001 JP 2001363385; 30.10.2002 WO PCT/JP02/11293
(43) Date of publication of application: 29.09.2004
(73) Proprietor: TOUDAI TLO, LTD., Tokyo 113- 0033 (JP)
(72) Inventor: Taira, Kazunari, Tsukuba-shi, Ibaraki 305-0046 (JP); Miyagishi, Makoto, Abiko-shi, Chiba 270-1166 (JP)
(74) Representative: Grünecker, Kinkeldey, Stockmair & Schwanhäusser Anwaltssozietät
(86) International application number: PCT/JP2002/012447
(87) International publication number: WO 2003/046186

(56) References cited:
- EP-A1- 1 371 727
- WO-A1-01/34815
- WO-A1-02/66638
- WO-A2-01/68836
- WO-A2-02/68635
- WO-A2-03/020931
- KENNERDELL JASON R ET AL: "Heritable gene silencing in Drosophila using double-stranded RNA" NATURE BIOTECHNOLOGY, NATURE PUBLISHING GROUP, NEW YORK, NY, US, vol. 18, no. 8, August 2000 (2000-08), pages 896-898, XP002184279 ISSN: 1087-0156
- TAVERNARAKIS N ET AL: "HERITABLE AND INDUCIBLE GENETIC INTERFERENCE BY DOUBLE-STRANDED RNAENCODED BY TRANSGENES" NATURE GENETICS, NATURE AMERICA, NEW YORK, US, vol. 24, February 2000 (2000-02), pages 180-183, XP002938530 ISSN: 1061-4036
- KAMATH R S ET AL: "Effectiveness of specific RNA-mediated interference through ingested double-stranded RNA in Caenorhabditis elegans." GENOME BIOLOGY, vol. 2, no. 1, 20 December 2000 (2000-12-20), pages 1-10, XP002411133 ISSN: 1465-6914 -& FRASER ANDREW G ET AL: "Functional genomic analysis of C. elegans chromosome I by systematic RNA interference" NATURE, NATURE PUBLISHING GROUP, LONDON, GB, vol. 408, no. 6810, 16 November 2000 (2000-11-16), pages 325-330, XP002199983 ISSN: 0028-0836
- LEE R C ET AL: "An extensive class of small RNAs in Caenorhabditis elegans" SCIENCE, AMERICAN ASSOCIATION FOR THE ADVANCEMENT OF SCIENCE,, US, vol. 294, no. 5543, 26 October 2001 (2001-10-26), pages 862-864, XP002284273 ISSN: 0036-8075
- PAUL C P ET AL: "EFFECTIVE EXPRESSION OF SMALL INTERFERING RNA IN HUMAN CELLS" NATURE BIOTECHNOLOGY, NATURE PUBLISHING GROUP, NEW YORK, NY, US, vol. 20, no. 5, May 2002 (2002-05), pages 505-508, XP001121066 ISSN: 1087-0156
- NAN SOOK LEE ET AL: "Expression of small interfering RNAs targeted against HIV-1 rev transcripts in human cells" NATURE BIOTECHNOLOGY, NATURE PUBLISHING GROUP, NEW YORK, NY, US, vol. 20, no. 5, May 2002 (2002-05), pages 500-505, XP002366543 ISSN: 1087-0156
- TUSCHL T: "Expanding small RNA interference" NATURE BIOTECHNOLOGY, NATURE PUBLISHING GROUP, NEW YORK, NY, US, vol. 20, no. 5, May 2002 (2002-05), pages 446-448, XP002232258 ISSN: 1087-0156
- PADDISON PATRICK J ET AL: "Short hairpin RNAs (shRNAs) induce sequence-specific silencing in mammalian cells" GENES AND DEVELOPMENT, COLD SPRING HARBOR LABORATORY PRESS, PLAINVIEW, NY, US, vol. 16, no. 8, 15 April 2002 (2002-04-15), pages 948-958, XP002204653 ISSN: 0890-9369
- MIYAGISHI MAKOTO ET AL: "Development and application of siRNA expression vector." NUCLEIC ACIDS RESEARCH. SUPPLEMENT (2001) 2002, no. 2, 2002, pages 113-114, XP002410667
- KAWASAKI H ET AL: "Identification of genes that function in the TNF-alpha-mediated apoptotic pathway using randomized hybrid ribozyme libraries" NATURE BIOTECHNOLOGY, NATURE PUBLISHING GROUP, NEW YORK, NY, US, vol. 20, no. 4, 23 April 2002 (2002-04-23), pages 376-380, XP002955698 ISSN: 1087-0156
- KASIM VIVI ET AL: "Control of siRNA expression using the Cre-loxP recombination system" NUCLEIC ACIDS RESEARCH, OXFORD UNIVERSITY PRESS, SURREY, GB, vol. 32, no. 7, 2004, page e66, XP002333060 ISSN: 0305-1048
- TAKI M ET AL: "SMALL-INTERFERING -RNA EXPRESSION IN CELLS FROM A DUMBBELL-SHAPED DNA" ANGEWANDTE CHEMIE. INTERNATIONAL EDITION, WILEY VCH VERLAG, WEINHEIM, DE, vol. 43, no. 24, 2004, pages 3160-3163, XP002981765 ISSN: 1433-7851
- ZANTA M A ET AL: "Gene delivery: a single nuclear localization signal peptide is sufficient to carry DNA to the cell nucleus" PROCEEDINGS OF THE NATIONAL ACADEMY OF SCIENCES OF USA, NATIONAL ACADEMY OF SCIENCE, WASHINGTON, DC, US, vol. 96, no. 1, January 1999 (1999-01), pages 91-96, XP002104949 ISSN: 0027-8424
- MIYAGISHI M. & TAIRA K.: 'U6 promoter-driven siRNAs with four uridine 3' overhangs efficiently suppress targeted gene expression in mammalian cells' NATURE BIOTECHNOLOGY vol. 20, no. 5, May 2002, pages 497 - 500, XP002961100
- BRUMMELKAMP T.R. ET AL.: 'A system for stable expression of short interfering RNAs in mammalian cells' SCIENCE vol. 296, no. 5567, 19 April 2002, pages 550 - 553, XP002225638
- SUI GUANGCHAO ET AL.: 'A DNA vector-based RNAi technology to suppress gene expression in mammalian cells' PROC. NATL. ACAD. SCI. USA vol. 99, no. 8, 16 April 2002, pages 5515 - 5520, XP002964701
- YU JENN-YAH ET AL.: 'RNA interference by expression of short-interfering RNAs and hairpin RNAs in mammalian cells' PROC. NATL. ACAD. SCI. USA vol. 99, no. 9, 30 April 2002, pages 6047 - 6052, XP002204654
- WANG Z. ET AL.: 'Inhibition of trypanosoma brucei gene expression by RNA interference using an integratable vector with opposing T7 promoters' J. BIOL. CHEM. vol. 275, no. 51, 22 December 2000, pages 40174 - 40179, XP002964702
- OHKAWA J. & TAIRA K.: 'Control of the functional activity of an antisense RNA by a tetracycline-responsive derivative of the human U6 snRNA promoter' HUM. GENE THER. vol. 11, 2000, pages 577 - 585, XP000926522
- LEWANDOSKI M. ET AL.: 'Zp3-cre, a transgenic mouse line for the activation or inactivation of loxP-flanked target genes specifically in the female germ line' CURRENT BIOLOGY vol. 7, no. 2, 1997, pages 148 - 151, XP002094596
- MARIA FE C MEDINA AND SADHNA JOSHI: "RNA-polymerase III-driven expression cassettes in human gene therapy" CURRENT OPINION IN MOLECULAR THERAPEUTICS, vol. 1, no. 5, October 1999 (1999-10), pages 580-594, XP001055319

## Description

### Technical Field

The present invention relates to an *in vivo* siRNA expression system capable of silencing the target gene expression, and a method for producing knock-down cells using this expression system.

### Background Art

RNA interference (hereafter abbreviated as "RNAi") is the phenomenon (process) capable of inducing the degradation of target gene mRNA so as to silence the target gene expression by introducing into cells a double-stranded RNA (hereafter abbreviated as "dsRNA") that comprises a sense RNA having the sequence homologous to the target gene mRNA and antisense RNA having the sequence complementary to the sense RNA. RNAi, because of its capability to silence the target gene expression, has received considerable attention as a simple gene knock-down method that replaces the conventional gene disruption method relying on the tedious, inefficient homologous recombination, or as a means ofgene therapy. The above-mentioned RNAi was originally discovered in nematodes (Fire, A. et al. Potent and specific genetic interference by double-stranded RNA in Caenorhabditis elegans. Nature 391, 806-811 (1998)). Thereafter, it is also observed in various organisms including plants, round worms, *Drosophila,* and protozoa (Fire, A. RNA-triggered gene silencing. Trends Genet. 15, 358-363 (1999) ; Sharp, P. A. RNA interference 2001. Genes Dev. 15, 485-490 (2001); Hammond, S. M., Caudy, A. A. & Hannon, G. J. Post-transcriptional gene silencing by double-stranded RNA. Nature Rev. Genet. 2, 110-119 (2001); Zamore, P. D. RNA interference: listening to the sound of silence. Nat Struct Biol. 8, 746-750 (2001) ) . Silencing of target gene expression was confirmed by actually introducing exogenous dsRNA in these organisms. This technique has been employed as a method for producing knock-down individuals.

Similar to in these organisms , RNAi induction in mammalian cells has been attempted by introduction of exogenous dsRNA. However, in this case, protein synthesis was inhibited by the action of host's protective mechanisms against the virus infection which was triggered by the transfected dsRNA, so that RNAi could not be observed.

Recently, Tuschl et al. reported that RNAi can be induced also in mammalian cells by transducing the cells with short dsRNAs of 21 or 22 nucleotide long having a single-stranded 2 or 3 nucleotide 3' overhang in place of long dsRNAs as those used in other organisms (Elbashir, S. M. et al. Duplexes of 21-nucleotide RNAs mediate RNA interference in cultured mammalian cells. Nature 411, 494-498 (2001) ; Caplen, N. J. et al. Specific inhibition of gene expression by small double-stranded RNAs in invertebrate and vertebrate systems. Proc. Natl. Acad. Sci. U S A 98, 9742-9747 (2001) ) .

As described above, RNAi has also been successfully induced in mammalian cells using small interfering double-stranded RNA (hereafter abbreviated as "siRNA") . For the functional analysis and gene therapy based on the gene silencing by RNAi, an efficient introduction of siRNA into cells and its stable intracellular maintenance become essential.

Efficiency in introducing exogenous siRNAs into cells varies depending on the cell type, being as low as 1% to 10% in certain cells. Also, exogenous siRNAs introduced into mammalian cells disappear in a few days after introduction, having no sufficient stability required for analysis of gene functions. Furthermore, in gene therapy, administration of siRNA at regular intervals becomes necessary, which increases physical loads of patients.

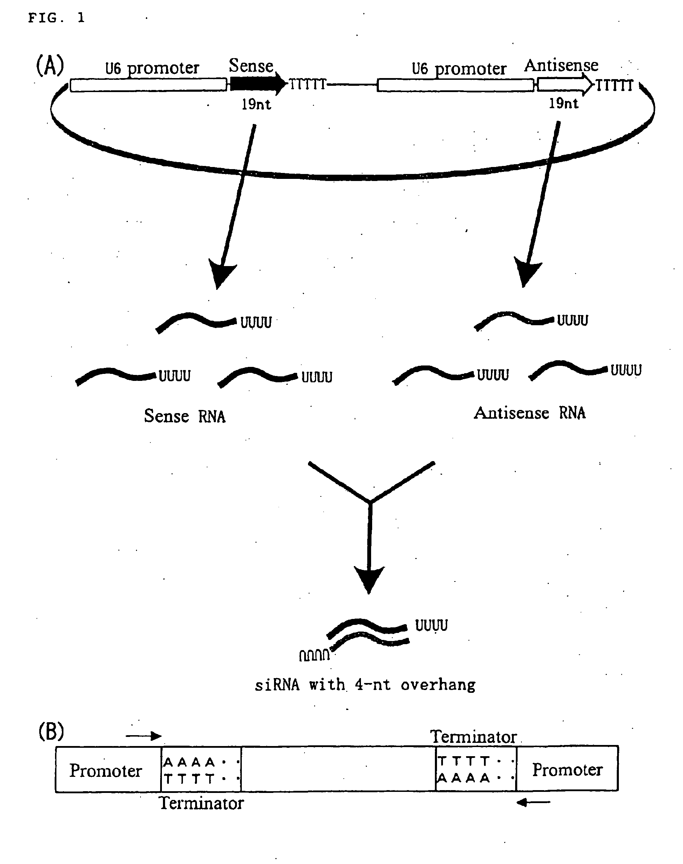

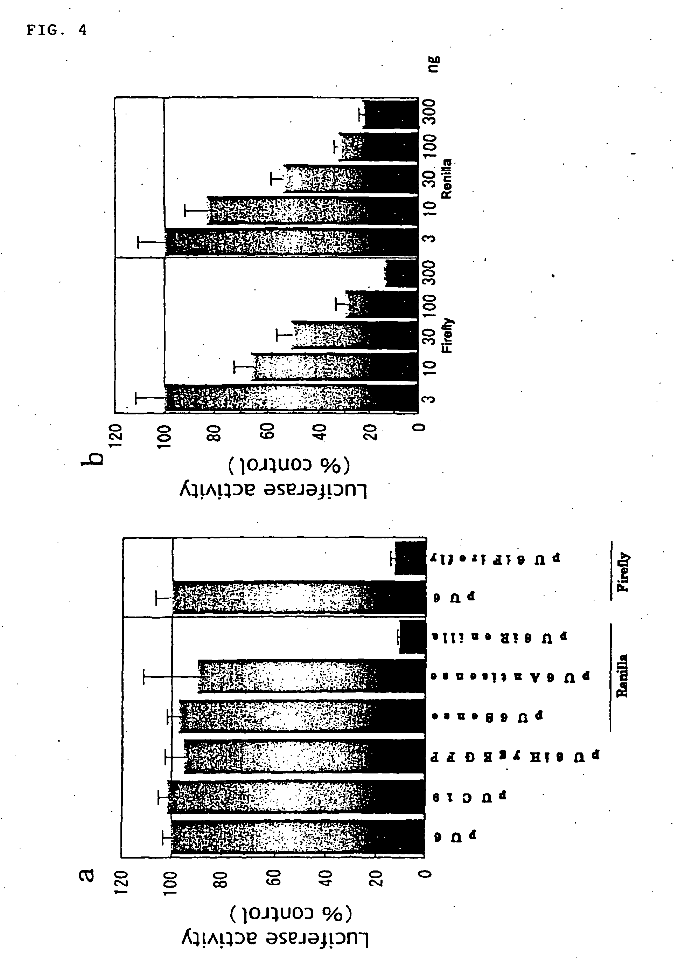

Moreover, it is extremely difficult to induce RNAi exclusively in a specific tissue or at a specific stage of development/differentiation by introduction of exogenous siRNA. In addition, though siRNAs are small in size, synthesis of RNA is markedly so expensive compared to DNA synthesis and the RNAi induction directly by siRNA is not economical.
Wang, Z. et al. (J. Biol. Chem. 275 (2000), pp. 40174-9) discloses a tetracycline-inducible expression system for dsRNA for gene silencing in *Typanosoma brucei* using opposing T7 Promoters. The dsRNAs used by Wang, Z. *et al.* have a size of more than 500 bp.
Ohkawa, J. & Taira, K. (Hum. Gene Ther. 11 (2000), pp. 577-85) disclose an expression system for controlling the functional activity of antisense RNA in HeLa cells using the U6 promoter (pol III promoter).
Paddison, P. J. et al. (Genes Dev. 16 (2002), 15. April, pp. 948-58) describe gene silencing in mammalian cells via short hairpin RNAs (shRNAs). The constructs have a double stranded region of 19-29 bp and are driven by a U6 promoter (Fig. 4a).
Tuschl, T. (Nature Biotechnol. 20 (2002), May, pp. 446-8) summarizes vector systems capable of producing small interfering RNAs (siRNAs) for downregulating gene expression in mammalian cells. U6 and H1 pol III promoters are disclosed for driving the expression of shRNAs that are processed into siRNA duplexes (e.g. Fig. 1).
Paul, C. P. et al. (Nature Biotechnol. 20 (2002), May, pp. 505-8) report on the expression of siRNA in human cells using a U6 promoter driven cassette. The laminin gene sequence constructs employed for the silencing are small hairpin RNAs.
Sui, G. et al. (PNAS 99 (2002), 16. April, pp. 5515-20) disclose an expression system for inducing RNAi in mammalian cells. The expression system is based on DNA vectors that contain shRNA with 21 bp double stranded region controlled by an U6 promoter (Fig.1).
WO 01/68836 relates to a method for attenuating gene expression by introducing an artificially synthesized dsRNA into cells that were made more responsible to RNAi by the expression or activation of enzymes necessary for RNAi function (e.g. Dicer and/or Argonaut). The size of the artificially synthesized dsRNAs is at least 50 bp, preferably between 400-800 bp (page 3).
Medina, M.F.C. & Joshi, S. (Curr. Op. Mol. Ther. 1, No. 5 (1999), pp. 580-94) disclose RNA polymerase III-promoter driven expression cassettes in human gene therapy.
US 60/317,229 relates to the intracellular expression of small interfering polyribonucleic acid molecules (siRNAs) and discloses let-7 hybrid constructs, or transfection with externally produced siRNA (siRNA constructs in pUC19, *in vitro* transcribed from a T7 promoter). The siRNAs used consist of (i) a 18-22 bp sequence that targets an mRNA and (ii) a loop structure covalently linked to (i), where the loop preferably has the nucleotide sequence of the let-7 loop.
WO 02/068635 relates to a method of inhibiting expression of a target gene by exposing a mammalian cell *in vitro* to a chemically synthesized double stranded RNA.
EP 1371727 discloses a method for introducing dsRNA in such a way that RNAi effect is sustained in mammalian (mainly mouse) cells for a long period of time. EP1371727 discloses the use of pol II promoters such as EF-1 alpha promoter.
Kennerdell et al. (Nature Biotech., Vol.18, p.896-898, 2000) disclose systems for gene silencing in Drosophila comprising T7 based in vitro RNA sense and antisense expression or transgenic vectors expressing a hairpin-loop RNA.
Tavernarakis et al. (Nature Genetics, Vol.24, p.180-183, 2000) provides a vector, pPD49.78, which comprises a sense and antisense DNA connected in the opposite directions and expressed under inducible hsp16-2 promoter and discloses said interfering RNA expression system in nematodes.
Kamath et al. (Genome Biology, Vol.2(1), p.1-10, 2000) provides an siRNA library expression system, comprising a library of dsRNA expressing bacteria for a *C*. *elegans* screen, said expression is carried out through a vector containing a target sequence and two T7 promoters inducible with IPTG.
WO 01/034815 discloses the interfering RNA expression system in insects under the control of baculovirus IE-1 promoter.
Lee et al. (Science, Vol.294, p.862-864, 2001) discloses miRNA sequences in *C*. *elegans* which are natural small sequences that form stem loop structures and refers also expression of such sequences in mammalian cells.

Now that most of the primary DNA sequence of the human genome has been determined, systematic and efficient methods for searching for functional genes has been developed to speedily elucidate gene functions. Gene silencing by RNAi can be utilized for the systematic search for the functional gene based on the phenotypic alteration of cells or individuals to accelerate the finding and analysis of novel functional genes.

### Disclosure of the Invention

An objective of the present invention is to provide an intracellular siRNA expression system capable of producing RNAi more efficiently, stably, and economically in cells, a method for producing knock-down cells using this siRNA expression system, and a method for searching for functional genes using this siRNA expression system.

In view of the above-mentioned problems, the present inventors studied the *in vivo* siRNA expression system and succeeded in its development. More specifically, the present invention relates to:
(1) An intracellular siRNA expression system,
   wherein said siRNA comprises a double strand region in which an antisense RNA strand and a sense RNA strand complementary to the antisense RNA pair up, and is directed against a region of a target gene mRNA in a mammalian cell, and
   wherein said antisense and sense RNA strands are produced from a vector comprising a DNA encoding said antisense RNA strand and a DNA encoding said sense RNA strand, and one or more pol III promoters capable of producing said antisense and sense RNA strands,
   wherein said vector comprises any one of the following (a) to (c):
   a) a stem-loop siRNA producing unit comprising a DNA encoding the antisense RNA strand and a DNA encoding the sense RNA strand complementary to the antisense RNA strand which are linked in the opposite direction via a linker, and a pol III promoter capable of producing said stem-loop siRNA at either side of said unit, wherein the double-stranded RNA region of the siRNA contains 1 to 7 mismatches and/or 1 to 7 bulges, and wherein one of nucleotides in the mismatch is guanine, and the other is uracil,
   b) a double-stranded DNA region in which a DNA encoding the antisense RNA strand and a DNA encoding the sense RNA strand complementary to the antisense RNA strand pair up, and two pol III promoters facing to each other with said double-stranded DNA region in between which are capable of producing said antisense and sense RNA strands, or
   c) a DNA encoding the antisense RNA strand, a DNA encoding the sense RNA strand complementary to the antisense RNA strand, and pol III promoters at the 5' ends of the antisense and sense code DNAs which are capable of producing said antisense and sense RNA strands.
(2) The siRNA expression system according to (1), wherein the double strand region of the siRNA expressed by the system is 15 to 49 bp long.
(3) The siRNA expression system according to (1), wherein the double strand region of the siRNA expressed by the system is 15 to 35 bp long.
(4) The siRNA expression system according to (1), wherein the double strand region of the siRNA expressed by the system is 15 to 30 bp long.
(5) The siRNA expression system according to any one of (1) to (4), wherein said pol III promoter is U6 promoter.
(6) The siRNA expression system according to any one of (1) to (5), wherein said promoter is an inducible promoter.
(7) The siRNA expression system according to any one of (1) to (6), wherein said system comprises loxP sequences in the form of any one of the following (a) to (c) so that the expression can be controlled:
   (a) the promoter comprises distal sequence element (DSE) and proximal sequence element (PSE) with a space therebetween, and in the space two loxP sequences, one in the vicinity of DSE and the other in the vicinity of PSE;
   (b) the promoter comprises DSE and PSE that are located to maintain the promoter activity, a loxP sequence therebetween, and another loxP sequence either upstream of DSE or downstream of PSE; and
   (c) two loxP sequences are located so as to interpose the DNAs encoding an antisense RNA and a sense RNA.
(8) The siRNA expression system according to (1), wherein said system comprises loxP sequences in the form of any one of the following (a) to (d) so that the expression can be controlled:
   (a) the promoter comprises DSE and PSE with a space therebetween, and in the space two loxP sequences, one in the vicinity of DSE and the other is the vicinity of PSE;
   (b) the promoter comprises DSE and PSE that are located to maintain the promoter activity, a loxP sequence therebetween, and another loxP upstream of DSE or downstream of PSE;
   (c) two loxPs are located so as to interpose the DNAs encoding an antisense RNA and a sense RNA; and
   (d) two loxPs are arranged so as to interpose a linker comprising a stop sequence.
(9) The siRNA expression system according to any one of (1) to (8), wherein said vector is a plasmid vector.
(10) The siRNA expression system according to any one of (1) to (8), wherein said vector is a viral vector.
(11) The siRNA expression system according to any one of (1) to (8), wherein said vector is a dumbbell-shaped DNA vector.
(12) A transformed cell comprising the siRNA expression system according to any one of (1) to (11).
(13) The cell according to (12), wherein said cell is a mammalian cell.
(14) A composition comprising the siRNA expression system according to any one of (1) to (11).
(15) The composition according to (14), wherein said composition is a pharmaceutical composition.
(16) A method for producing *in vitro* a cell in which the expression of a target gene mRNA is silenced, wherein said method comprises the steps of: introducing the siRNA expression system according to any one of (1) to (11) into cells, and selecting cells in which said siRNA expression system is introduced.
(17) An intracellular siRNA library expression system which produces plural kinds of the siRNA according to any one of (1) to (11)in a mammalian cell.
(18) The siRNA library expression system according (17), wherein siRNAs expressed by the system are composed of random RNA strands.
(19) The siRNA library expression system according to (17), wherein said system is an assembly of multiple siRNA expression vectors that each targets a gene sequence comprising a coding region and/or a non-coding region.
(20) The siRNA library expression system according to (17), wherein siRNAs expressed by the system are composed of RNA strands encoded by DNA fragments of any cDNA or genomic DNA, said fragment has the length of the siRNA to be expressed.
(21) An assembly comprising plural kinds of the siRNA library expression systems according to any one of (17) to (20), wherein different siRNAs are expressed by each system in said assembly.
(22) A method of searching for a functional gene, the method comprising the steps of:
   (a) introducing the siRNA library expression system according to any one of (17) to (20) or the assembly of plural kinds of the siRNA library expression systems according to (21) into cells;
   (b) selecting cells into which said siRNA library expression system or said assembly has been introduced; and
   (c) analyzing the phenotype of the cells thus selected.
(23) The method of searching for a functional gene according to (22), wherein said method further comprising a step of screening for a functional gene based on the sequence of DNA coding for siRNA in the cell whose phenotype has been found altered as the result of the phenotype analysis.
(24) A method for selecting a highly active siRNA, the method comprising the steps of:
   (a) introducing the siRNA library expression system according to any one of (17) to (20), or the assembly of plural kinds of the siRNA library expression systems according to (21) into cells, and
   (b) measuring the expression level of a specific gene or protein in the cells into which said siRNA library expression system or said assembly is introduced.

In one aspect, the present invention relates to an intracellular siRNA expression system. This siRNA expression system comprises an antisense code DNA coding for the antisense RNA directed against a region of the target gene mRNA, a sense code DNA coding for the sense RNA directed against the same region of the target gene mRNA, and one or more promoters capable of expressing the antisense and sense RNAs from the antisense and sense code DNAs, respectively.

"siRNA" means a small interfering RNA that is a short-length double-stranded RNA that are not toxic in mammalian cells. The length is not limited to 21 to 23 bp long as reported by Tuschl, et al. (ibid) . There is no particular limitation in the length of siRNA as long as it does not show toxicity. "siRNAs" can be, for example, 15 to 49 bp, preferably 15 to 35 bp, and more preferably 21 to 30 bp long. Alternatively, the double-stranded RNA portion of a final transcription product of siRNA to be expressed can be, for example, 15 to 49 bp, preferably 15 to 35 bp, and more preferably 21 to 30 bp long. The double-stranded RNA portions of siRNAs in which two RNA strands pair up are not limited to the completely paired ones, and may contain nonpairing portions due to mismatch (the corresponding nucleotides are not complementary) , bulge (lacking in the corresponding complementary nucleotide on one strand) , and the like. Nonpairing portions can be contained to the extent that they do not interfere with siRNA formation. The "bulge" used herein preferably comprise 1 to 2 nonpairing nucleotides, and the double-stranded RNA region of siRNAs in which two RNA strands pair up contains preferably 1 to 7, more preferably 1 to 5 bulges. In addition, the "mismatch" used herein is contained in the double-stranded RNA region of siRNAs in which two RNA strands pair up, preferably 1 to 7, more preferably 1 to 5, in number. In a preferable mismatch, one of the nucleotides is guanine, and the other is uracil. Such a mismatch is due to a mutation from C to T, G to A, or mixtures thereof in DNA coding for sense RNA, but not particularly limited to them. Furthermore, in the present invention, the double-stranded RNA region of siRNAs in which two RNA strands pair up may contain both bulge and mismatched, which sum up to, preferably 1 to 7, more preferably 1 to 5in number.

Such nonpairing portions (mismatches or bulges, etc.) can suppress the below-described recombination between antisense and sense code DNAs and make the siRNA expression system as described below stable. Furthermore, although it is difficult to sequence stem loop DNA containing no nonpairing portion in the double-stranded RNA region of siRNAs in which two RNA strands pair up, the sequencing is enabled by introducing mismatches or bulges as described above. Moreover, siRNAs containing mismatches or bulges in the pairing double-stranded RNA region have the advantage of being stable in *Escherichia coli* or animal cells.

The terminal structure of siRNA may be either blunt or cohesive (overhanging) as long as siRNA enables to silence the target gene expression due to its RNAi effect. The cohesive (overhanging) end structure is not limited only to the 3' overhang as reported by Tuschl et al. (ibid.), and the 5' overhanging structure may be included as long as it is capable of inducing the RNAi effect. In addition, the number of overhanging nucleotide is not limited to the already reported 2 or 3, but can be any numbers as long as the overhang is capable of inducing the RNAi effect. For example, the overhang consists of 1 to 8, preferably 2 to 4 nucleotides. Herein, the total length of siRNA having cohesive end structure is expressed as the sum of the length of the paired double-stranded portion and that of a pair comprising overhanging single-strands at both ends. For example, in the case of 19 bp double-stranded RNA portion with 4 nucleotide overhangs at both ends, the total length is expressed as 23 bp. Furthermore, since this overhanging sequence has low specificity to a target gene, it is not necessarily complementary (antisense) or identical (sense) to the target gene sequence. Furthermore, as long as siRNA is able to maintain its gene silencing effect on the target gene, siRNA may contain a low molecular weight RNA (which may be a natural RNA molecule such as tRNA, rRNA or viral RNA, or an artificial RNA molecule), for example, in the overhanging portion at its one end.

In addition, the terminal structure of the "siRNA" is necessarily the cut off structure at both ends as described above, and may have a stem-loop structure in which ends of one side of double-stranded RNA are connected by a linker RNA. The length of the double-stranded RNA region (stem-loop portion) can be, for example, 15 to 49 bp, preferably 15 to 35 bp, and more preferably 21 to 30 bp long. Alternatively, the length of the double-stranded RNA region that is a final transcription product of siRNAs to be expressed is, for example, 15 to 49 bp, preferably 15 to 35 bp, and more preferably 21 to 30 bp long. Furthermore, there is no particular limitation in the length of the linker as long as it has a length so as not to hinder the pairing of the stem portion. For example, for stable pairing of the stem portion and suppression of the recombination between DNAs coding for the portion, the linker portion may have a clover-leaf tRNA structure. Even though the linker has a length that hinders pairing of the stem portion, it is possible, for example, to construct the linker portion to include introns so that the introns are excised during processing of precursor RNA into mature RNA, thereby allowing pairing of the stem portion. In the case of a stem-loop siRNA, either end (head or tail) of RNA with no loop structure may have a low molecular weight RNA. As described above, this low molecular weight RNA may be a natural RNA molecule such as tRNA, rRNA or viral RNA, or an artificial RNA molecule.

The term "target gene" refers to a gene whose expression is silenced due to siRNA to be expressed by the present system, and can be arbitrarily selected. As this target gene, for example, genes whose sequences are known but whose functions remain to be elucidated, and genes whose expressions are thought to be causative of diseases are preferably selected. A target gene may be one whose genome sequence has not been fully elucidated, as long as a partial sequence of mRNA of the gene having at least 15 nucleotides or more, which is a length capable of binding to one of the strands (antisense RNA strand) of siRNA, has been determined. Therefore, genes expressed sequence tags (ESTs) and a portion of mRNA, of which have been elucidated, may be selected as the "target gene" even if their full lengths has not been determined.

"Antisense RNA" is an RNA strand having a sequence complementary to a target gene mRNA, and thought to induce RNAi by binding to the target gene mRNA. "Sense RNA" has a sequence complementary to the antisense RNA, and annealed to its complementary antisense RNA to form siRNA. These antisense and sense RNAs have been conventionally synthesized with an RNA synthesizer. In the present invention, these RNAs are intracellularly expressed from DNAs coding for antisense and sense RNAs (antisense and sense code DNAs) respectively using the siRNA expression system.

To express antisense and sense RNAs from the antisense and sense code DNAs respectively, the siRNA expression system of the present invention comprises "promoter." The type, number and location of the promoter can be arbitrarily selected as long as it is capable of expressing antisense and sense code DNAs. As a simple construction of siRNA expression system, a tandem expression system can be formed, in which a promoter is located upstream of both antisense and sense code DNAs. This tandem expression system is capable of producing siRNAs having the aforementioned cut off structure on both ends. In the stem-loop siRNA expression system (stem expression system) , antisense and sense code DNAs are arranged in the opposite direction, and these DNAs are connected via a linker DNA to construct a unit. A promoter is linked to one side of this unit to construct a stem-loop siRNA expression system. Herein, there is no particular limitation in the length and sequence of the linker DNA, which may have any length and sequence as long as its sequence is not the termination sequence, and its length and sequence do not hinder the stem portion pairing during the mature RNA production as described above. As an example, DNA coding for the above-mentioned tRNA and such can be used as a linker DNA.

In both cases of tandem and stem-loop expression systems, the 5' end may be have a sequence capable of promoting the transcription from the promoter. More specifically, in the case of tandem siRNA, the efficiency of siRNA production may be improved by adding a sequence capable of promoting the transcription from the promoters at the 5' ends of antisense and sense code DNAs. In the case of stem-loop siRNA, such a sequence can be added at the 5' end of the above-described unit. A transcript from such a sequence may be used in a state of being attached to siRNA as long as the target gene silencing by siRNA is not hindered. If this state hinders the gene silencing, it is preferable to perform trimming of the transcript using a trimming means (for example, ribozyme as described below) .

In either case of the above-mentioned tandem or stem expression system, either pol II or pol III promoter may be used as long as it is capable of producing the corresponding RNAs from the above-described DNAs. Preferably, a pol III promoter suitable for expressing short RNAs such as siRNAs can be used. Pol III promoters include the U6 promoter, tRNA promoter, retroviral LTR promoter, Adenovirus VA1 promoter, 5Sr RNA promoter, 7SK RNA promoter, 7SL RNA promoter, and H1 RNA promoter. The U6 promoter adds four uridine nucleotides to the 3' end of RNA, thus the 3' overhang of the finally produced siRNA can be freely made to be of 4, 3, 2, 1, or 0 nucleotide by providing the 5' end sequence of the antisense and sense code DNAs with 0, 1, 2, 3 or 4 adenines. In the case of using other promoters, the number of 3' overhanging nucleotide can be freely altered.

In the case of using pol III promoters, it is preferable to further provide a terminator at 3' ends of sense and antisense code DNAs in order to express only the short RNAs and suitably terminate the transcription. Any terminator sequence can be used as long as it is capable of terminating the transcription initiated by the promoter. A sequence consisting of four or more consecutive adenine nucleotides, a sequence capable of forming the palindrome structure, etc. can be used.

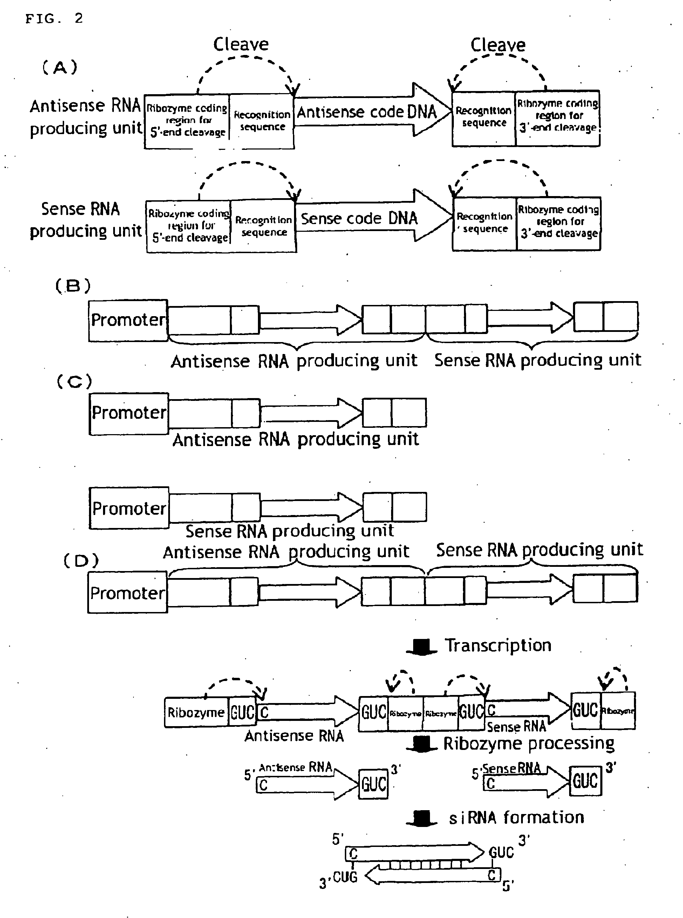

Pol II promoters include the cytomegalovirus promoter, T7 promoter, T3 promoter, SP6 promoter, RSV promoter, EF-1α promoter, β-actin promoter, γ-globulin promoter, and SRα promoter. A pol II promoters produce not short RNAs as in the case of a pol III promoter, but somewhat longer RNAs. Therefore, when pol II promoters are used, it is necessary to produce antisense or sense RNA by truncating somewhat longer RNA using a means to cleave RNA by self-processing such as a ribozyme. A unit for producing antisense or sense RNA using a ribozyme may have the following construction. As shown in Fig. 2A, the antisense or sense RNA producing unit has the antisense or sense code DNA, the regions coding for the RNA sequence recognized by the ribozyme (recognition sequence coding region) at its 5' and 3'-ends, and the regions coding for the 5'- and 3'-end cleaving ribozymes to cut off recognition sequences, which regions are arranged outward adjacent to the respective recognition sequence coding regions. Such antisense and sense RNA producing units may be operatively linked in tandem downstream of the same pol II promoter (Fig. 2B), or separately downstream of the respective promoters (Fig. 2C). Although Fig. 2B shows an example where both units are linked in tandem, if necessary, it is also possible to insert an arbitrary spacer sequence between the antisense and sense RNA producing units so as to adjust the distance between the two RNAs expressed by the respective units so that a ribozyme can readily act on the RNAs.

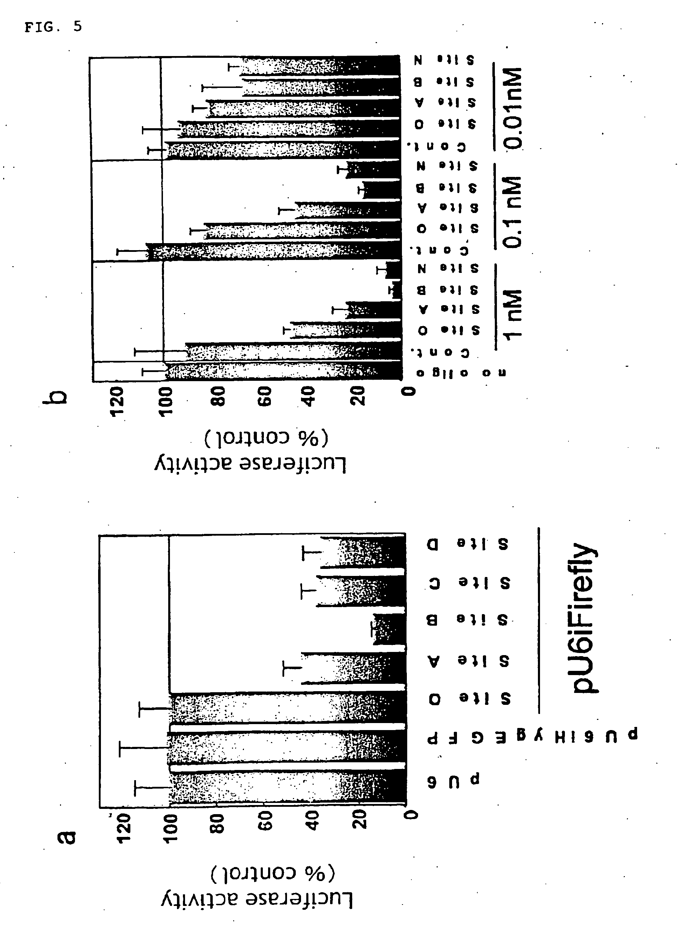

The ribozymes that cleaves 5'- and 3'-ends of the antisense and sense code DNAs may be a hammerhead ribozyme (Biochem. Biophys. Res. Commun., Vol. 186, pp.1271-1279 (1992); Proc. Natl. Acad. Sci. USA, Vol. 90, pp.11302-11306 (1993)). The hammerhead ribozymes may have any other sequences so long as they are capable of self-processing (BIO medica, Vol. 7, pp.89-94 (1992)). Also, ribozymes are not limited to the hammerhead ones, and, for example, the hairpin ribozyme, HDV ribozyme, and *Tetrahymena*-derived ribozyme may be used as long as they are capable of self-processing (Gene, Vol. 122, pp.85-90 (1992)). The ribozyme recognition sequences are those recognized by the 5'- and 3'-cleaving ribozymes. For example, the hammerhead ribozyme cleaves the phosphodiester linkage of NUH sequence (N is A, G, C or U, while H is A, C, or U. Although any of nucleotide combinations may be used, "GUC" is preferred as the most efficiently cleaved sequence at its 3'-side. Therefore, when the hammerhead ribozyme is used, NUH, preferably GUC can be used as a recognition sequence. An example of the mRNA construction for producing antisense and sense RNAs is shown in Fig. 2D, in which a combination of this hammerhead ribozyme and the recognition sequence GUC is added to the 5'- and 3'-ends of antisense and sense RNAs. In Fig. 2D, 5'-ends of antisense and sense RNAs are made to be "C" in order to form the 2-nucleotide overhang. When the 3-nucleotide overhang is to be formed, the 5'-end is not limited to "C." In the construction shown in Fig. 2D, the sequence GUC is added to the 3'-side.

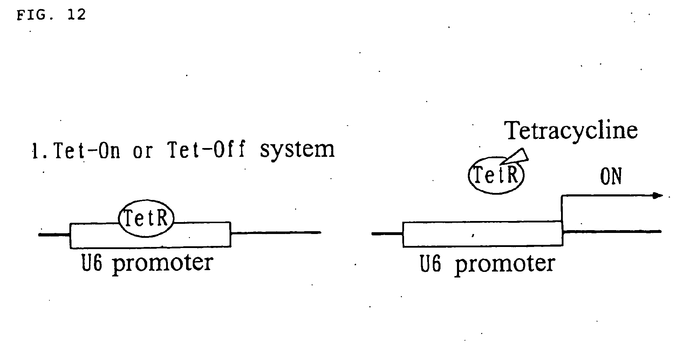

If an inducible promoter is used as the promoter in this invention, siRNA can be expressed at any desired timing. Such inducible promoters include the tetracycline-inducible U6 promoter (Ohkawa, J. & Taira, K. Control of the functional activity of an antisense RNA by a tetracycline-responsive derivative of the human U6 snRNA promoter. Hum. Gene Ther. 11, 577-585 (2000); Fig. 12). In addition, siRNA expression may be tissue-specifically induced using a tissue-specific promoter or a DNA recombination system such as Cre-loxP system.

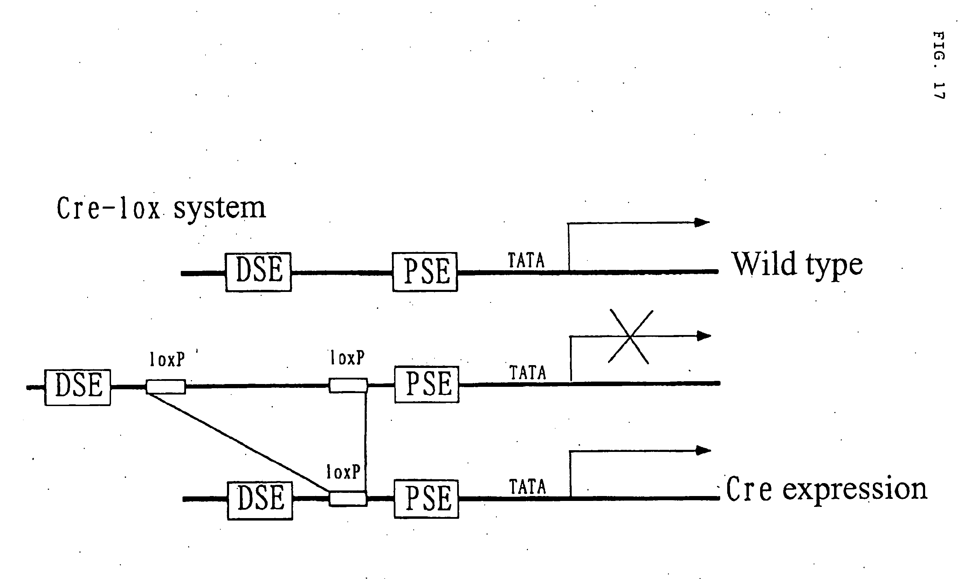

Moreover, instead of using a promoter inducible by drugs and such as described above, it is possible to control the siRNA production using, for example, a recombinase. A case of using the CRE-loxP recombinase system will be described as an example (Fig. 17). In the promoter, Distal Sequence Element (DSE) and Proximal Sequence Element (PSE) are located with a space therebetween , and in the space a loxP sequence is arranged in the vicinity of DSE and another loxP sequence in the vicinity of PSE. Usually, due to a distance between DSE and PSE, the promoter activity is in the *off* state so as to inhibit the siRNA expression. The action of CRE protein on this expression system induces recombination between loxP sequences located in the vicinities of DSE and PSE, resulting in the displacement of DNA between loxP sequences. Then, DSE and PSE come close to each other to turn the promoter activity to the *on* state for expressing siRNA. This example describes an siRNA producing system in which the promoter activity is turn to the *on* state by the action of CRE. In contrast, it is also possible to construct a system inhibiting the siRNA expression by the action of CRE (not shown). For example, one loxP is provided between DSE and PSE that are arranged so as to maintain the promoter activity, and another loxP is located either upstream of DSE or downstream of PSE. In the absence of Cre protein, the promoter activity is in the *on* state, and, in its presence, DSE or PSE is displaced through recombination between loxPs to turn the promoter activity to the *off* state, leading to the suppression of siRNA production. Although this is an example in which loxP is arranged in the promoter region, it is also possible to provide two loxPs so as to interpose the antisense or sense code DNA to suppress the siRNA production by supplying the CRE protein.

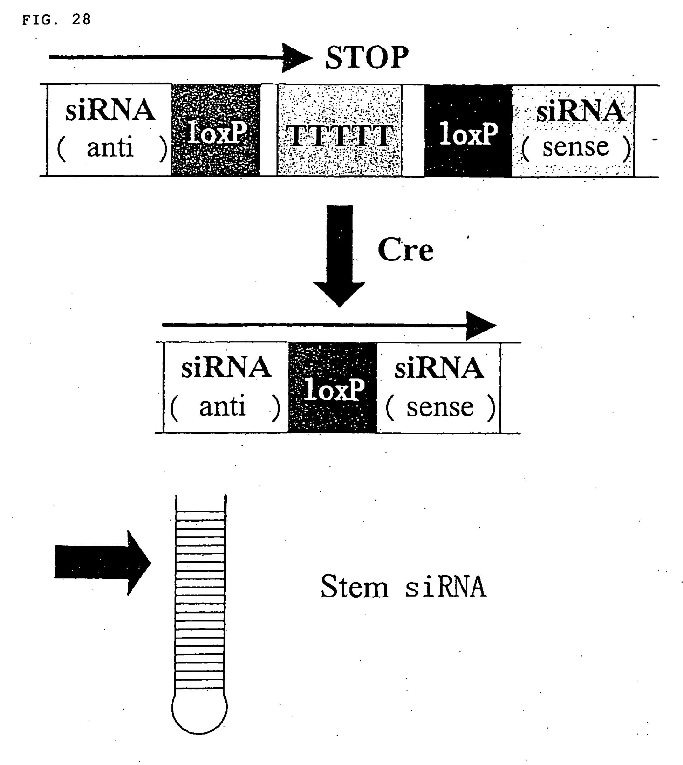

Furthermore, in the case of the stem-loop siRNA expression system, it is possible to provide two loxPs in the linker portion so as to interpose the stop sequence (e.g. TTTTT). Without CRE protein, transcription from the promoter is terminated at the stop sequence in the linker portion, leading to the suppression of siRNA production. CRE protein induces the recombination between loxPs to displace the stop sequence, leading to transcription of antisense and sense code DNAs to produce the stem-loop siRNA (cf. Fig. 28).

The siRNA expression system comprising the abovementioned "promoter," "antisense code DNA" and "sense code DNA" can be integrated as such into the chromosome to intracellularly express antisense and sense RNAs, thereby producing siRNA. Preferably, the siRNA expression system is introduced into the target such as cells using a vector carrying the expression system to efficiently transfer the system. The vector usable in this invention can be selected depending on the target to be transfected, such as cells, and includes, for mammalian cells, viral vectors such as retrovirus vector, adenovirus vector, adeno-associated virus vector, vaccinia virus vector, lentivirus vector, herpesvirus vector, alphavirus vector, EB virus vector, papilloma virus vector, and foamyvirus vector, and non-viral vectors including cationic liposome, ligand DNA complex, gene gun, etc. (Y. Niitsu, et al., Molecular Medicine 35: 1385-1395 (1998)), but not limited to them. It is also possible to preferably use, instead of viral vectors, the dumbbell-shaped DNA (Zanta M.A. et al., Gene delivery: a single nuclear localization signal peptide is sufficient to carry DNA to the cell nucleus. Proc Natl Acad Sci U S A. 1999 Jan 5; 96 (1) : 91-6) , DNA modified so as to have nuclease resistance, or naked plasmids (Liu F, Huang L. Improving plasmid DNA-mediated liver gene transfer by prolonging its retention in the hepatic vasculature. J. Gene Med. 2001 Nov-Dec; 3 (6) : 569-76). The present inventors, as shown in the Examples described below, found it possible to efficiently silence the expression of target gene by maintaining the siRNA expression system of this invention in a dumbbell-shaped DNA. Therefore, in a preferred embodiment of the present invention, the siRNA expression system maintained in a dumbbell-shaped DNA molecule is preferably used. The dumbbell-shaped DNA can be linked to antibody, peptide, and such to facilitate its introduction into cells.

The antisense and sense RNAs may be expressed in the same vector or in different vectors. For example, the construction for expressing both antisense and sense RNAs from the same vector can be prepared by linking a promoter, such as a pol III promoter capable of expressing short RNA, upstream of antisense and sense code DNAs to form antisense and sense RNA expression cassettes, and inserting these cassettes into a vector either in the same direction or opposite directions. An example of such a construction, in which these cassettes are inserted in the same direction, is shown in Fig. 1A. It is also possible to construct an expression system, as shown in Fig. 1B, in which antisense and sense code DNAs are arranged on different strands in the opposite orientation so as to pair up. This construction may comprise one double-stranded DNA comprising antisense and sense RNA coding strands (DNA coding for siRNA) , and promoters on both sides facing to each other so as to express the antisense and sense RNAs from the respective DNA strands. In this case, to avoid the addition of excess sequences downstream of the sense and antisense RNAs, it is preferable to place a terminator at 3' ends of the respective strands (strands coding for antisense and sense RNAs). The terminator may be a sequence of four or more consecutive adenine (A) nucleotides. In this palindrome expression system, it is preferable to use two different promoters. Herein, in these expression systems, as shown in Fig. 1A, siRNAs with the cut off structure at both ends are produced.

Furthermore, as an alternative construction capable of expressing the above-described stem-loop siRNAs, it is also possible to form a unit in which both antisense and sense code DNAs are arranged in the opposite orientation on the same DNA strand via a linker, and link the resulting unit downstream of a single promoter. In this case, the order of expression is not necessarily limited to "DNA coding for antisense RNA -> linker -> DNA coding for sense RNA," but may be "DNA coding for sense RNA -> linker -> DNA coding for antisense RNA." RNA produced by the expression system of this type construction has a stem-loop structure in which the linker portion forms a loop and sense and antisense RNAs on its both sides pair up (a stem structure). Then, the loop portion in this palindrome is cleaved by intracellular enzymes to produce the siRNA. In this case, the length of the stem portion, the length and type of the linker, and such can be selected as described above.

In a system using the ribozyme as shown in Fig. 2, the system represented in Fig. 2B may be inserted into a vector, or two cassettes shown in Fig. 2C may be inserted into the same vector either in the same direction or opposite directions. It is also possible to maintain a system capable of expressing a plurality of siRNAs (siRNAs directed against different target gene mRNAs, siRNAs directed against different target sites of the same target gene mRNA, or siRNA directed against the same target site of the same target gene mRNA) in a single vector.

The system for expressing antisense and sense RNAs in different vectors may be constructed by linking, for example, a pol III promoter capable of expressing short RNAs, upstream of the antisense and sense code DNAs to construct antisense and sense RNA expression cassettes, and introducing these cassettes into different vectors. Furthermore, the expression system using the ribozyme can be constructed by introducing two cassettes as shown in Fig. 2C in different vectors.

If required, it is also possible to allow a vector to further carry a sequence that enables selecting cells transfected with the vector, such as a selection marker. Examples of selection markers include a drug resistance marker such as the neomycin resistance gene, hygromycin resistance gene, and puromycin resistance gene, markers that can be selected based on the enzyme activity as an indicator such as galactosidase, markers selectable by fluorescence emission as an indicator such as GFP, markers that can be selected with the cell surface antigen such as EGF receptor, B7-2, and CD4 as an indicator, etc. The selection marker enables selecting only the cell transfected with the vector, namely, the cell transfected with the siRNA expression system. Therefore, a low transfection efficiency in the conventional transfer of exogenous siRNA fragments into cells can be improved, and only cells expressing siRNA can be concentrated. Furthermore, the use of vector can prolong the period maintaining the siRNA expression system. Vectors such as the retrovirus vector induce the integration of the system into chromosomes, enabling stable supply of siRNA from the siRNA expression system in the cells.

The present invention relates to cells maintaining the above-mentioned siRNA expression system. Cells to be transduced with this siRNA expression system are preferably mammalian cells because siRNA is capable of inducing RNAi in mammalian cells, in which RNAi has been conventionally difficult to be induced. Furthermore, cells which are difficult to maintain a long-term stable expression of long-chain dsRNAs, such as plant cells, may be used as the cells to be transduced with the present siRNA expression system.

Methods for introducing the above-mentioned siRNA expression system into the above-described cells may be arbitrarily selected depending on cells. For example, for the transduction of mammalian cells, the method may be selected from the calcium phosphate method (Virology, Vol. 52, p.456 (1973)), electroporation (Nucleic Acids Res., Vol. 15, p.1311 (1987)), lipofection (J. Clin. Biochem. Nutr., Vol. 7, p.175 (1989)), viral infection-mediated method (Sci. Am., p.34, March (1994)), gene gun method, etc. Transduction of plant cells can be carried out by the electroporation (Nature, Vol . 319, p. 791 (1986)), polyethylene glycol method (EMBO J., Vol. 3, p. 2717 (1984)), particle gun method (Proc. Natl. Acad. Sci. USA, Vol. 85, p.8502 (1988)), *Agrobacterium*-mediated method (Nucleic Acids Res., Vol. 12, p 8711 (1984)), etc.

Selection of cells transduced with the above-described siRNA expression system may be carried out by known techniques such as hybridization and PCR using DNA sequence specific for the siRNA expression system as a probe or primer. However, when the siRNA expression system is maintained in the vector provided with a selection marker, the selection can be performed with the phenotype owing to the marker as an indicator.

Cells transduced with the siRNA expression system become knock-down cells in which the target gene expression is silenced. Herein, "knock-down cells" include cells in which the target gene expression is completely suppressed, and those not completely suppressed but reduced. Knock-down cells have been conventionally produced by deleting or modifying a target gene or its regulatory region.. In contrast, the use of the siRNA expression system according to the present invention enables to simply produce cells in which the target gene expression is suppressed by introducing the siRNA expression system into cells and selecting the transduced cells without any modification of the target gene on chromosomes. The knock-down cells according to the present invention can be used as research tools for the functional analysis of a target gene, and cells in which a disease-causative gene as the target has been silenced can be used as disorder model cells and such. Furthermore, target gene knock-down animals, disorder model animals and so on can be produced by introducing the above-described siRNA expression system into germ cells, and generating individual organisms from the germ cells maintaining the system.

There is no particular limitation in the method of producing target gene knockdown animals using the above-described siRNA expression system, and any known method may be used. As an example, the siRNA expression vector is injected into fertilized eggs obtained by mating an F1 female mouse (e.g. CBA/JxC57BL/6J) with a male mouse (e.g. C57BL/6J). The peripheral blood DNA is obtained from the tail of a mouse developed from the above-mentioned fertilized egg, and subjected to genomic Southern blot analysis using a portion of the expression vector as a probe to identify the positive progenitor animal in which the siRNA expression vector has been integrated into its chromosomes. Backcrossing of the above-mentioned progenitor mouse with either C57BL/6J or F₁ (CBA/JxC57BL/6J) hybrid mouse is repeated to obtain their offspring mice. Then, genomic Southern blot and PCR analyses are performed to identify offsprings positive for the gene recombination.

Furthermore, although the case of introducing siRNA expression system mainly into mammals has been described above, the system may be used in plants. The RNAi induction by the direct introduction of conventional double-stranded RNA into plant cells is difficult to maintain RNAi effects due to the loss of dsRNA during the cell passage processes. It possible to maintain RNAi effect in plant cells by using the RNA expression system of the present invention to integrate the siRNA generation system into chromosomes in plant cells. It is also possible to create from these cells a transgenic plant that stably maintains RNAi effect. The transgenic plant can be created by methods known to those skilled in the art.

The present invention also relates to a composition containing the above-described siRNA expression system. Since the present siRNA expression system is capable of suppressing the expression of any desired target gene using siRNA, this system enables disorder-causative gene silencing. The siRNA expression system can be used as a pharmaceutical composition and such supplemented with appropriate vehicles.

Another embodiment of the present invention relates to a system for intracellularly expressing an siRNA library. siRNAs expressed by "siRNA library" of the present invention are composed of RNA strands comprising adenine, guanine, cytosine or uracil in any order and having a length of siRNA to be expressed or those encoded by (random) cDNA or genomic DNA fragments having a length of siRNA to be expressed. Herein, such siRNAs as described above are also referred to as "random siRNA." That is, "random siRNAs" used herein is composed of any sequences, or any sequences selected from specific cDNA sequences, sequences contained in a specific cDNA library, or genome sequences. The above-described siRNA expression system is capable of silencing a specific target gene expression, while the system of this embodiment can be used to search for novel functional genes by expressing an siRNA library and silencing arbitrary genes, for example, whose functions and sequences are unknown. An example of the siRNA library expression system has a construction as shown in Fig. 1B. This system comprises DNA coding for a double-stranded siRNA in which DNA coding for random antisense RNA and DNA complementary to the DNA coding for sense RNA are paired (hereinafter called "siRNA code DNA"), and two promoters that faces each other interposing the siRNA code DNA and are capable of separately expressing antisense RNA or sense RNA.

The above-described "random siRNAs" are the same as the above-mentioned siRNA expression system, except that they contains any sequences, or any sequences selected from specific cDNA sequences, sequences included in a specific cDNA library, or genomic sequences, and composed of double-stranded RNAs of such short strands as expressing no toxicity in mammalian cells. The short strand is not limited to 21 to 23 bp long as reported by Tuschl et al. (ibid) , and may be, for example, 15 to 49 bp, preferably 15 to 35 bp, and more preferably 21 to 30 bp long as long as it does not exhibit toxicity. In addition, the end structure of the above-mentioned random siRNAs may be either blunt or cohesive (overhanging) as long as they are capable of silencing the target gene by RNAi effect. In addition, the cohesive (overhanging) end structure may include not only the 3'-overhang but also 5'-overhang as long as it is capable of inducing the above-mentioned RNAi effect. Moreover, the number of overhanging nucleotide is not limited to 2 or 3, but may be any number capable of inducing RNAi effect, for example, 1 to 8 nucleotides, preferably 2 to 4 nucleotides. Furthermore, as described above, siRNA may comprise a low molecular RNA at the overhang on its one end. Moreover, as mentioned above, siRNA expressed by the siRNA library expression system may comprise a mismatch or a bulge, or both of them in the double-stranded RNA region in which RNAs pair up.

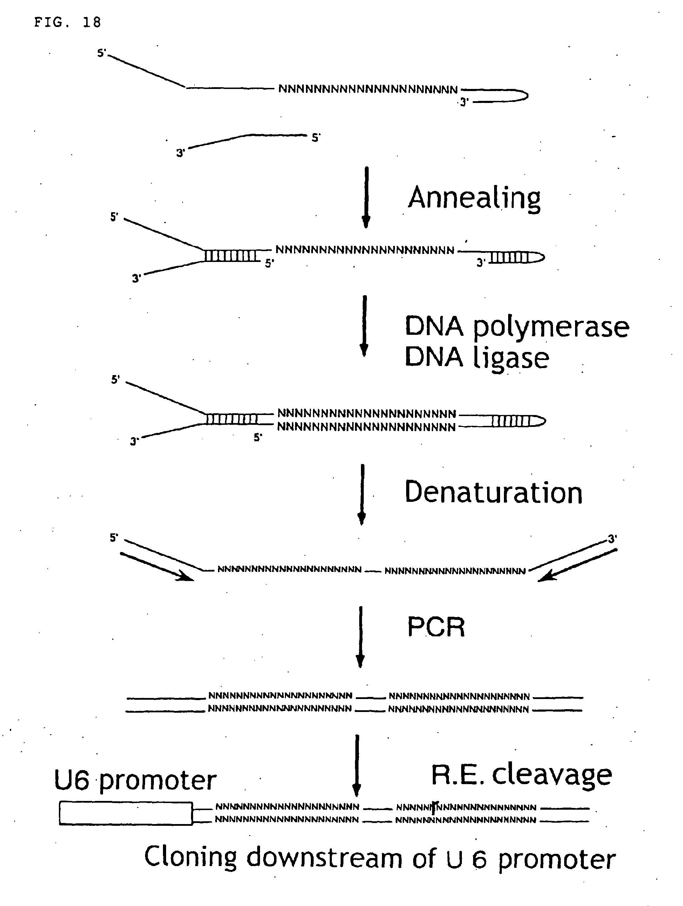

In addition, the siRNA library expression system is not limited to the above-described construction (having two promoters facing each other interposing the siRNA code DNA), and may have a construction capable of expressing the stem-loop siRNA. That is, the present invention also includes a construction in which a promoter is linked upstream of a unit (hereafter referred to as "stem-loop siRNA library producing unit) formed by connecting a DNA coding for an antisense RNA (for example, any random sequences, or any sequences selected from specific cDNA sequences, sequences included in a specific cDNA library, or genome sequences), and a DNA coding for sense RNA complementary to the above-mentioned antisense RNA in the opposite direction via a linker DNA. One example of a method of preparing the above-described stem-loop siRNA library producing unit is shown in Fig. 18. That is, a single-stranded DNA comprising a DNA coding for antisense RNA having a random sequence (antisense code DNA) and at its 3' end a sequence capable of forming an arbitrary palindrome structure, is synthesized using a DNA synthesizer or the like. A primer complementary to the 5' side of this single-stranded DNA is prepared, and annealed to it to form a palindrome structure at the 3' end of the single-stranded DNA. DNA polymerase and DNA ligase is allowed to act on this construction to synthesize the sense code DNA strand complementary to the antisense code DNA, and, at the same time, to form a palindrome structure in which a stem portion is elongated. This palindrome structure is made single-stranded by the denaturing treatment, and PCR is performed using primers complementary to the sequences at both sides of antisense code DNA and sense code DNA to produce a double-stranded DNA containing the stem-loop siRNA library producing unit. If necessary, one strand of the double-stranded DNA containing the stem-loop siRNA library producing unit is trimmed with restriction enzyme or the like, and the double-stranded DNA thus obtained is linked downstream of an appropriate promoter to produce the stem-loop siRNA library expression system.

From the above-described stem-loop siRNA library expression system, the stem-loop siRNA is produced. In this stem-loop siRNA, as described above, the length of the double-stranded RNA portion to be produced (stem portion) can be, for example, 15 to 49 bp, preferably 15 to 35 bp, and more preferably 21 to 30 bp long. In addition, there is no particular limitation in the length and sequence of the linkers as long as they do not hinder the pairing of the stem portion, and a low molecular RNA such as clover leaf tRNA may be provided as a linker.

The above-mentioned "random antisense code DNA" is composed of any sequences, which may be arbitrarily selected, for example, from a group of sequences that is formed by any combination of four nucleotides "A, G, C, and T" and has the length of siRNA to be expressed. Alternatively, the "random antisense code DNA" is composed of any sequence selected from specific cDNA sequences contained in a specific cDNA library, or genome sequences. Promoters usable herein may be pol II or pol III promoter. It is preferable to use pol III promoters suitable for expressing short RNA such as siRNA. Furthermore, the two promoters may be identical or different, preferably different in view of the expression efficiency. Examples of pol II and pol III promoters usable in this case are the same as described above.

In addition, when a pol III promoter is used, to appropriately terminate the transcription after the expression of complementary short RNA, it is preferable to provide terminator between the promoter and DNA coding for siRNA as shown in Fig. 1B. A, sequence consisting of 4 or more consecutive adenines as shown in Fig. 1B, any terminators known to those skilled in the art, and such may be used.

When an inducible promoter is used, an siRNA library can be expressed at a predetermined timing. It is thus possible to analyze genes functioning at specific development/differentiation stages of organisms. Furthermore, the use of a promoter having tissue-specific transcriptional activity enables induction of tissue-specific expression of siRNA, thereby allowing the analysis of functional genes in a specific tissue. Inducible promoters and tissue-specific promoters usable in this case are the same as those described above. The above-described siRNA library expression system can be integrated into chromosomes of cells as a DNA insert. For efficient introduction into cells and such, the siRNA library expression system is preferably maintained in a vector. "Vectors" usable herein are the same as those described above. It is also possible to improve screening efficiency of functional genes by introducing the siRNA library expression system capable of expressing a plurality of siRNAs in a single vector. If necessary, a vector carrying the siRNA library expression system may further comprise a selection marker or the like. Selection markers usable in this case are the same as those described above. Thus, the use of selection markers enables selection of cells transfected with the vector carrying the siRNA library expression system, thereby improving screening efficiency of functional genes.

Another embodiment of the siRNA expression system of this invention is an siRNA library expression system that is an assembly of multiple siRNA expression vectors that each targets a gene sequence comprising a coding region and/or a non-coding region.

It is also possible to collect siRNA library expression systems capable of expressing different siRNAs and construct an assembly. For example, siRNA code DNAs and a stem-loop siRNA library expression system may be constructed so as to produce, as the siRNAs to be expressed from this assembly, RNA strands comprising sequences that are formed by any combination of four nucleotides "A, G, C and U" and have the length of siRNAs to be expressed. Alternatively, siRNA code DNAs may comprise any cDNA fragments or any sequences selected from sequences included in any cDNA libraries, or genome sequences. Thus, the use of an assembly comprising a plurality of siRNA library expression systems enables more efficient search for functional genes.

Using the above-described random siRNA library expression system or assembly of these siRNA library expression systems, a method of searching for functional genes can be performed by the steps of: introducing an siRNA library expression system or the above-mentioned assembly of siRNA library expression systems into cells, selecting the cells transduced with the above-described siRNA library expression system or assembly, and analyzing phenotypes of the cells thus selected.

As described above, methods for introducing the siRNA library expression system or the like into cells may vary depending on the kind of cells. Specifically, methods of its introduction into mammalian cells can be selected from the calcium phosphate method (Virology, Vol. 52, p.456 (1973)), electroporation method (Nucleic Acids Res., Vol. 15, p.1311 (1987)), lipofection method (J. Clin. Biochem. Nutr., Vol. 7, p.175 (1989)), virus infectious transduction method (Sci. Am. p.34, March (1994)), gene gun method and the like, while its introduction into plant cells can be carried out by the electroporation method (Nature Vol. 319, p.791 (1986)), polyethylene glycol method (EMBO J. Vol. 3, p.2717 (1984)), particle gun method (Proc. Natl. Acad. Sci. USA Vol. 85, p.8502 (1988)), method mediated by *Agrobacterium* (Nucleic Acids Res. Vol . 12, p.8711 (1984)) and the like.

When the siRNA library expression system or the like is introduced into a vector carrying a selection marker, cells transduced with the system or assembly can be selected by collecting the cells having the phenotype due to the selection marker. When a selection marker is not contained, transduced cells can be selected by detecting them with the known hybridization method, PCR, and such using the specific sequence that is common to the siRNA library expression system as a probe or primer.

After the selection of cells transduced with the above-described siRNA library expression system or assembly, the phenotype of these cells can be analyzed by comparing it to that of control cells transduced with no siRNA library expression system or assembly. These phenotypes are not limited to those expressed only on the cell surface, but include, for example, intracellular alterations, and such.

Cells judged to have altered phenotypes by the above-mentioned analysis would contain the siRNA library expression system capable of silencing any of functional genes. Therefore, to screen for functional genes, probes and primers are constructed based on the DNA sequence coding for siRNA contained in this cell and are used in hybridization or PCR to conduct cloning of functional genes. Database search for functional genes can also be performed based on the DNA sequence coding for siRNA.

Effects of the siRNA expression system of the present invention usually greatly vary depending on the position of the target site of the target gene. For example, in the case of targeting HIV, a high gene silencing effect of siRNA can be expected by targeting a priming site. Even in the case where a preferable target site is unknown, the siRNA library expression system of the present invention is effective. That is, the above-mentioned siRNA library expression system of this invention is extremely useful as a system of searching for the optimal target site of mRNA to be effectively degraded by siRNA. The present invention provides a method for selecting a highly active siRNA comprising the steps of: introducing the siRNA library expression system, or assembly of siRNA library expression systems of this invention into cells, and measuring expression levels of a specific gene or protein in the cells transduced with the siRNA library expression system or the assembly thereof. Measurement of expression levels of any desired gene or protein can be easily carried out by the methods known to those skilled in the art such as Northern blot hybridization or Western blot hybridization.

The cells in which the siRNA expression system and siRNA library expression system of this invention is introduced are not particularly limited to mammalian cells, but include cells of other animals, plants, yeast, fungi, etc.

The siRNA expression library of the present invention can be used to, for example, search for viral infection-associated genes. The siRNA expression library is introduced into cells the cells are infected with a virus, and surviving cells are examined, thereby easily identifying genes associated with this viral infection. The use of the siRNA expression library containing the human 40,000 cDNAs enables the identification of all the viral infection-associated genes. The randomized siRNA expression library or the siRNA expression library of genome fragments enables to identify genes other than cDNAs. These two libraries may be used in combination.

### Brief Description of the Drawings

Fig. 1 represents an siRNA expression system using the U6 promoter, and a method for producing siRNA employing the system. (A) shows an siRNA production process. Two U6 promoters produce sense and antisense short RNAs, with adding four uridines (Us) to 3'-ends of RNAs. Sense and antisense RNAs thus expressed are annealed to form the duplex of siRNAs with a 4-nucleotide 3' overhang. (B) shows a palindrome type siRNA expression system, comprising a double-stranded DNA coding for siRNA comprising sense and antisense code DNAs and promoters at both ends, from which sense and antisense RNAs are expressed.
Fig. 2 represents an example of the construction for producing an siRNA expression system using a ribozyme.
Fig. 3 represents the EGFP gene silencing effect of an siRNA expression system directed against EGFP when the system was introduced into cells expressing hygromycin/EGFP. Left side panels (A, D, G and J) show the expression of hygromycin/EGFP; middle panels (B, E, H and K) the expression of DsRed; right side panels (C, F, I and L) the results of merged expressions of hygromycin/EGFP and DsRed.
Fig. 4 represents the gene silencing effect of an siRNA expression system directed against either sea pansy *(Renilla)* or firefly luciferase when the system was introduced into HeLa S3 cells having the luciferase activity. In Fig. 4a, an ordinate value means luciferase activity of the cells, in which the siRNA expression system directed against the firefly luciferase was introduced, normalized based on the sea pansy luciferase activity, or the activity of the cells, in which the siRNA expression system directed against the sea pansy luciferase was introduce, normalized based on the firefly luciferase activity. Fig. 4b shows the concentration-dependent silencing effect of the siRNA expression systems on firefly or sea pansy luciferase activity when a varied amount of the siRNA expression system directed against each luciferase was introduced into cells.
Fig. 5 represents the gene silencing effect using a series of siRNAs or siRNA expression systems directed against different target sites on the same target gene (for firefly luciferase). Fig. 5a shows the gene silencing effect of siRNA expression vectors directed against various target sites when the vectors were introduced into cells. Fig. 5b represents the results obtained when the exogenous siRNAs directed against different target sites were directly introduced into cells at different concentrations.
Fig. 6a represents the gene silencing effect of the length of 3' overhang of siRNA. Fig. 6b shows the gene silencing effect of siRNA expression systems directed against two target genes or two target sites. In Fig. 6b, luciferase activity values were normalized based on the activity of β-galactosidase introduced as an internal control.
Fig. 7 represents the capability of the siRNA expression system to silence the endogenous β-catenin gene. Panels A, B, and C are for the group transduced with the siRNA expression vector directed against β-catenin (pHygEGFP/iβ-catenin) , while panels D, E, and F are for the group transduced with the empty vector (pHygEGFP). These groups were all stained with the anti-β-catenin antibody. Left side panels (A and D) represent the expression of Hygromycin/EGFP; middle panels (B and E) the expression of β-catenin; and right side panels (C and F) the merged image of these two expressions.
Fig. 8 represents the comparison of RNAi effects between the tandem and stem-loop siRNAs. siRNA expression vectors pU6tandem19 and pU6stem19 are tandem and stem-loop, respectively. Cont. represents the control (vacant vector).
Fig. 9 represents the gene silencing effects of various siRNA expression vectors.
Fig. 10 represents the gene silencing effects of siRNA expression vectors containing a cytomegalovirus-derived promoter (CMV promoter), and tRNA promoter.
Fig. 11 represents the RNAi induction effects of double-stranded siRNAs containing a mismatch, or a bulge.
Fig. 12 is a diagram describing the principle of Tet-ON system. In the absence of tetracycline, the tetracycline repressor protein binds to U6 promoter, resulting in the suppression of transcription, while, in its presence, the tetracycline repressor protein binds to it to be released from U6 promoter so as to initiate transcription.
Fig. 13 is a graph representing RNAi inducing effects of siRNA expression vector having the tetracycline-inducible promoter. U6Teti represents an siRNA expression vector containing the tetracycline operator sequence in U6 promoter, and U6i represents an siRNA expression vector not containing the sequence.
Fig. 14 is a diagram depicting automatic cleavages in the RNA transcript containing a trimming ribozyme.
Fig. 15 is a diagram depicting siRNA production by self-processing of trimming ribozyme. Nucleotide cleavages occur at the positions indicated by black arrowheads to produce siRNAs.
Fig. 16 is an electrophoretogram showing siRNA production by RNA self-processing. Bands corresponding to 21nt siRNA are indicated with an arrow.
Fig. 17 is a diagram showing an example of the construction for controlling siRNA expression using the Cre-lox system.
Fig. 18 is a diagram representing an example of the preparation of the stem-loop siRNA library expression system.
Fig. 19 is a diagram representing an example of the preparation of the siRNA library expression system.
   ① shows a random DNA fragment having the dephosphorylated blunt ends of 19 to 29 bp long.
   ② represents the random DNA fragment ① which is ligated with the 5'-phosphorylated hairpin type DNA linker 1 at its both ends.
Fig. 20 is a continuation of Fig. 19.
   ③ shows strand displacement from the Nick site by Bst DNA Polymerase.
   ④ represents the fragment ③ which is ligated with DNA linker 2.
Fig. 21 is a continuation of Fig. 20.
   ⑤ shows strand displacement from the Nick site by Bst DNA Polymerase.
   ⑥ represents the cleavage of ⑤ by AscI.
Fig. 22 is a continuation of Fig. 21.
   represents an siRNA library expression pre-library.
   ⑧ shows BspMI cleavage of the siRNA library expression pre-library. In the case of inserting the Loop sequence, TTCG, between the sense and antisense code DNAs, the cleavage proceeds to step ⑧-2 in Fig. 23.
   ⑨ represents the completed siRNA library expression system as a result of blunting by Klenow Fragment, removal of DNA linker 1, and self-ligation.

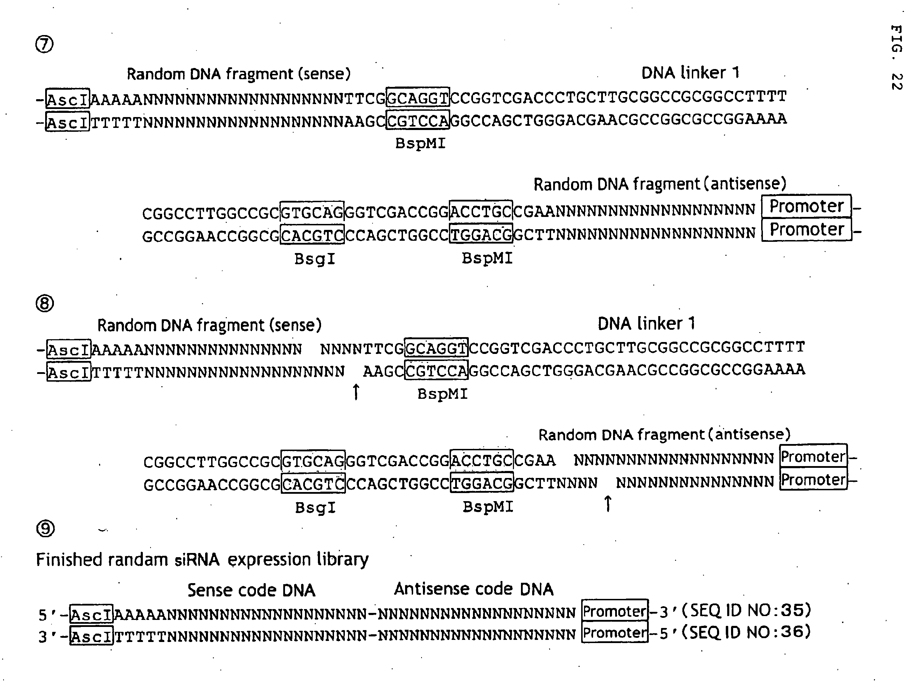

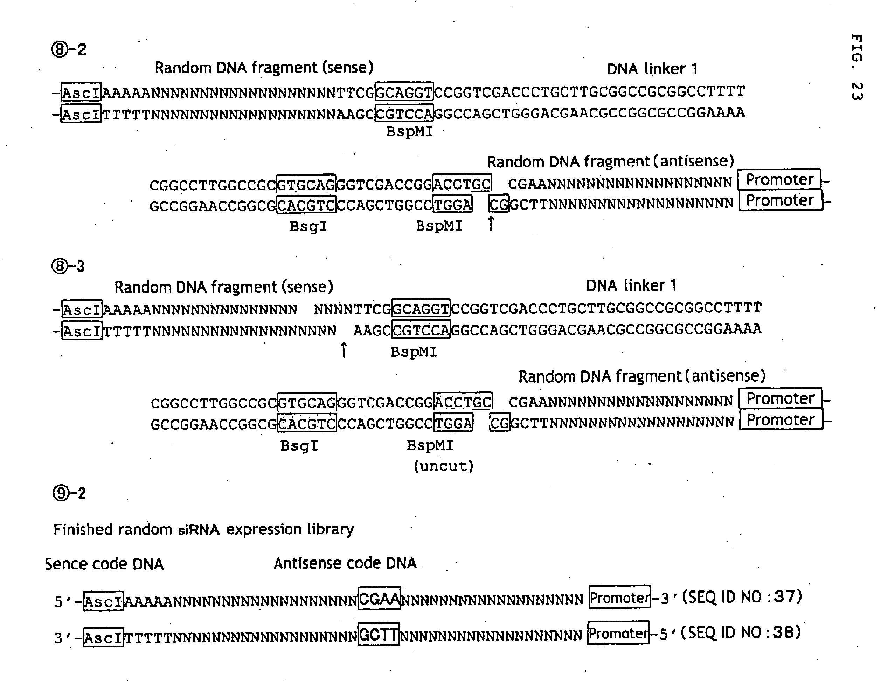

Fig. 23 is a continuation of Fig. 22.
   ⑧-2 represents the case of inserting the Loop sequence, TTCG, between the sense and antisense code DNAs in ⑧. The siRNA library expression pre-library is cleaved by BsgI.
   ⑧-3 shows cleavage of the siRNA library expression pre-library by BspMI. The cleaved site by BsgI cannot be attacked by BspMI.
   ⑨-2 represents the completed siRNA library expression system as a result of blunting by T4 DNA Polymerase, removal of DNA linker 1, and self-ligation.

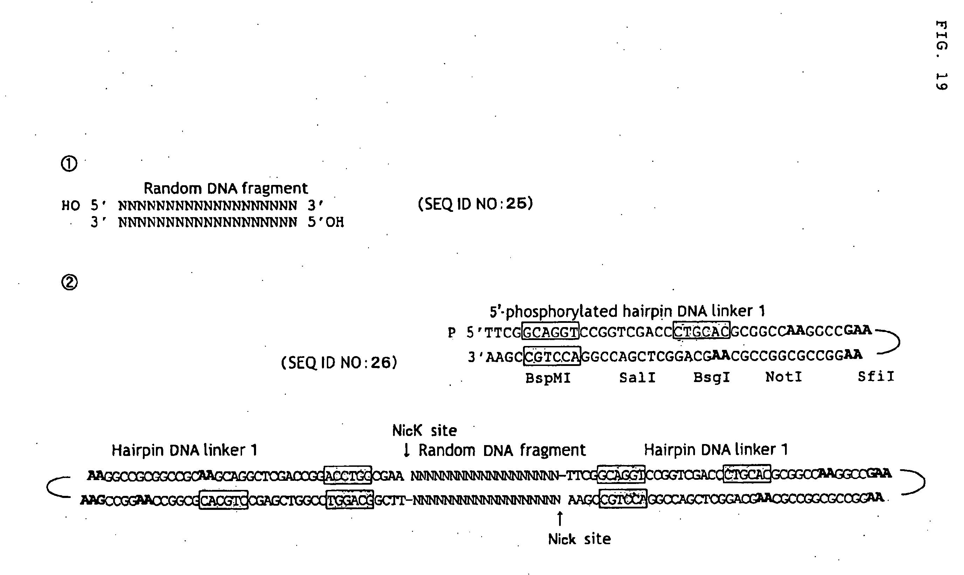

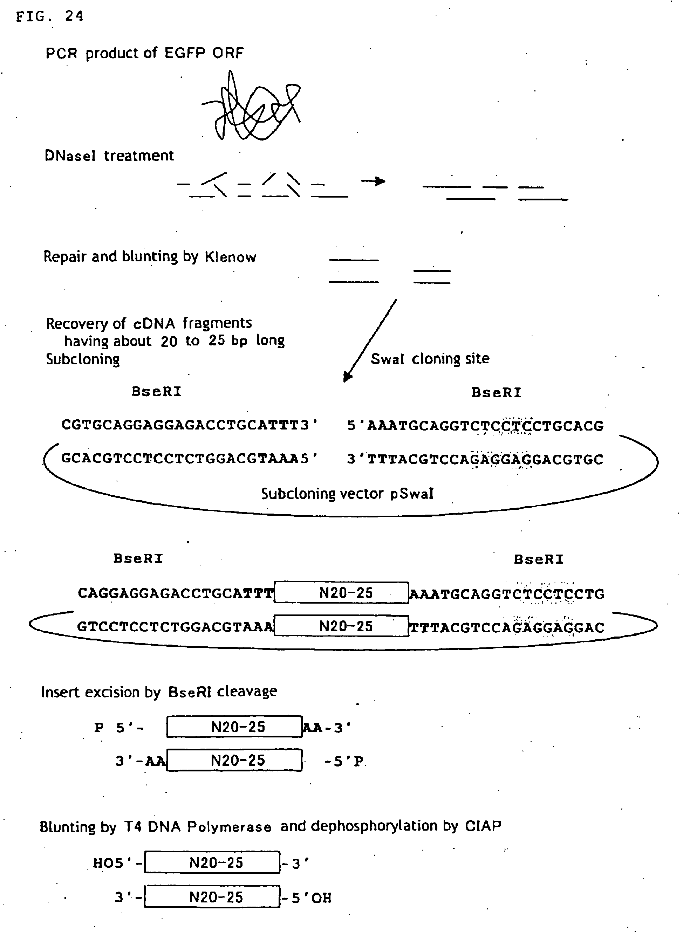

Fig. 24 is a diagram representing the preparation of EGFP cDNA fragment of approximately 20 to 25 bp long. The final product that is a random EGFP cDNA fragment of approximately 20 to 25 bp long with the dephosphorylated blunt end serves as the random DMA fragment in Fig. 19 ①.

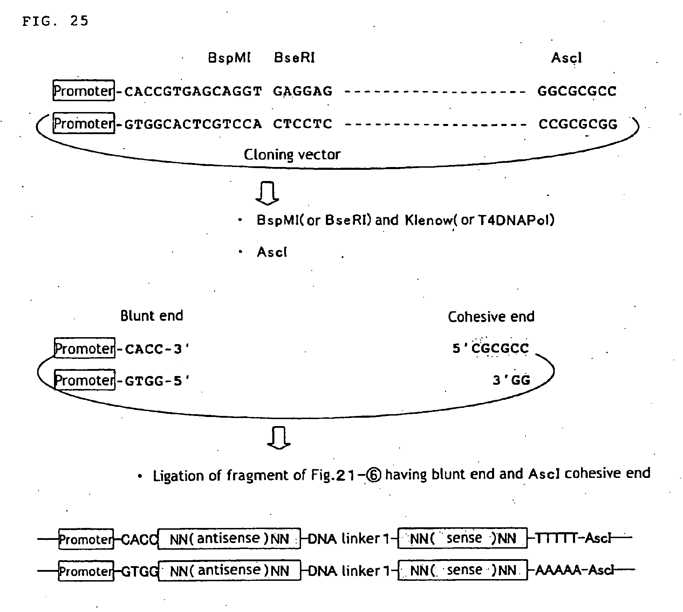

Fig. 25 is a diagram representing the preparation of a cloning vector. The promoter is either the human U6 promoter or human tRNA promoter. The cloning vector containing U6 promoter was prepared using BspMI and Klenow, while that containing tRNA promoter was prepared using BseRI and T4 DNA Polymerase.

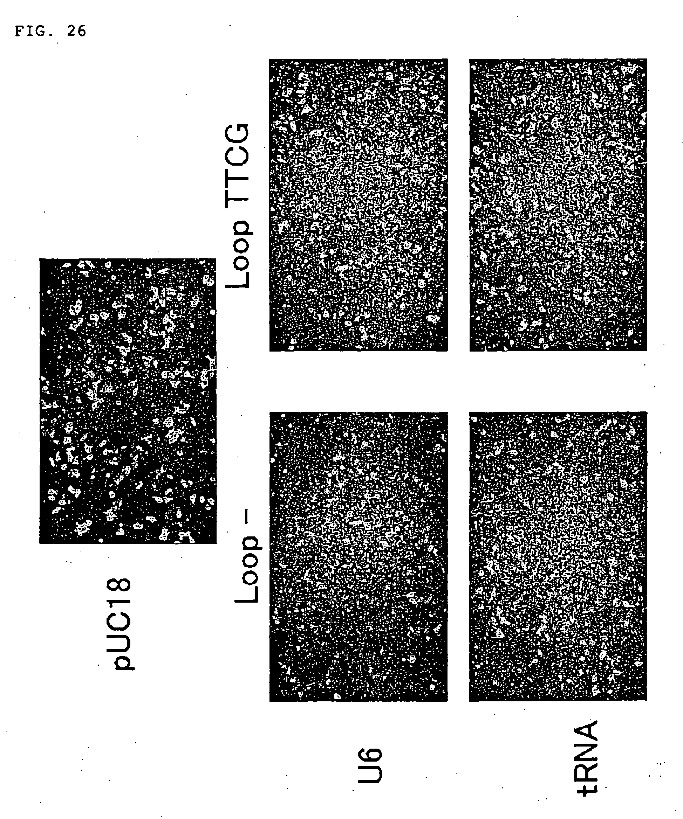

Fig. 26 represents micrographs showing the results of observing the EGFP fluorescence intensity with a confocal microscope.

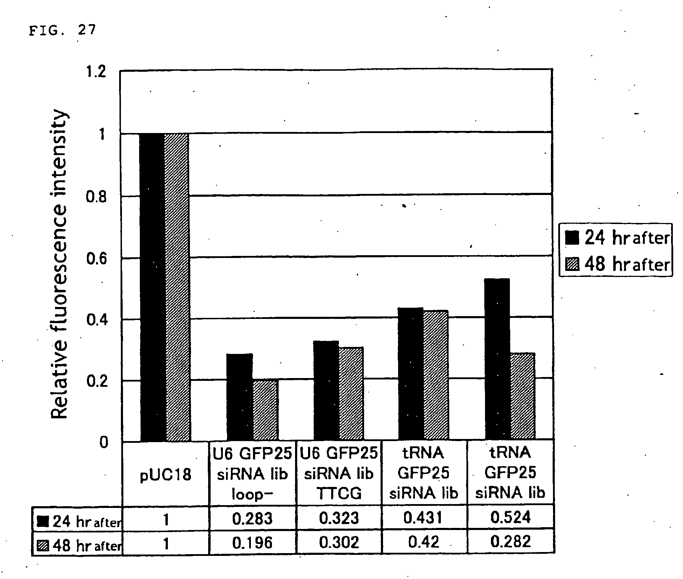

Fig. 27 is a graph representing relative EGFP fluorescence intensities in pUC18, U6 GFP25 siRNA lib-loop-, U6 GFP25 siRNA lib TTCG, tRNA GFP25 siRNA lib loop-, and tRNA GFP25 siRNA lib TTCG, measured 24 and 48 h after the transfection.
Fig. 28 is a diagram representing the stem-loop siRNA expression system containing two loxPs that interpose the linker portion containing the stop sequence.

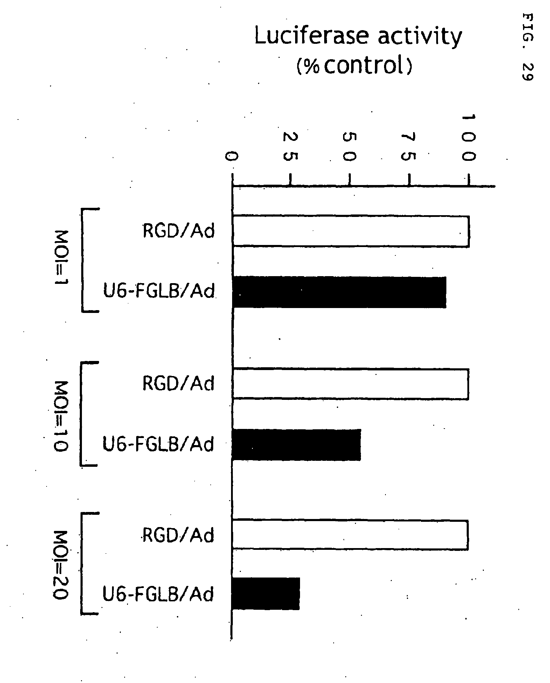

Fig. 29 is a graph representing the gene silencing effect of the siRNA expression adenovirus vector.

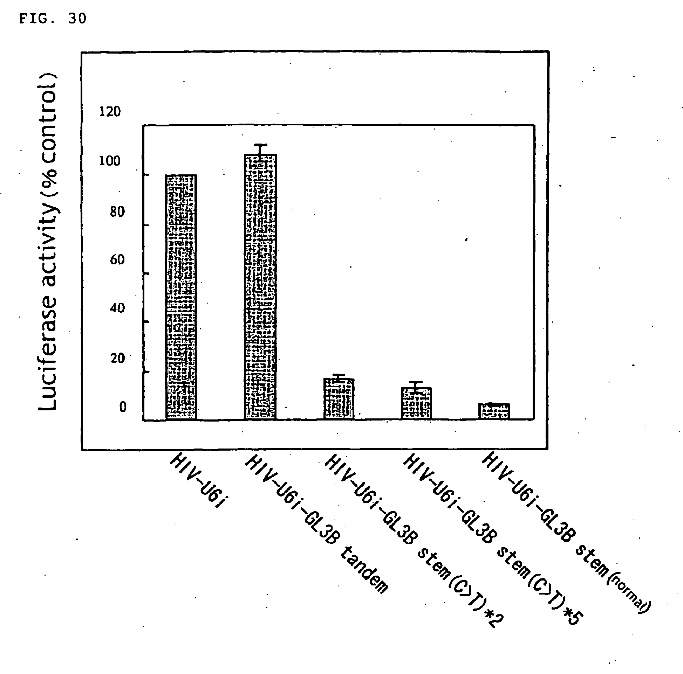

Fig. 30 is a graph representing the gene silencing effect of the siRNA expression HIV vector.

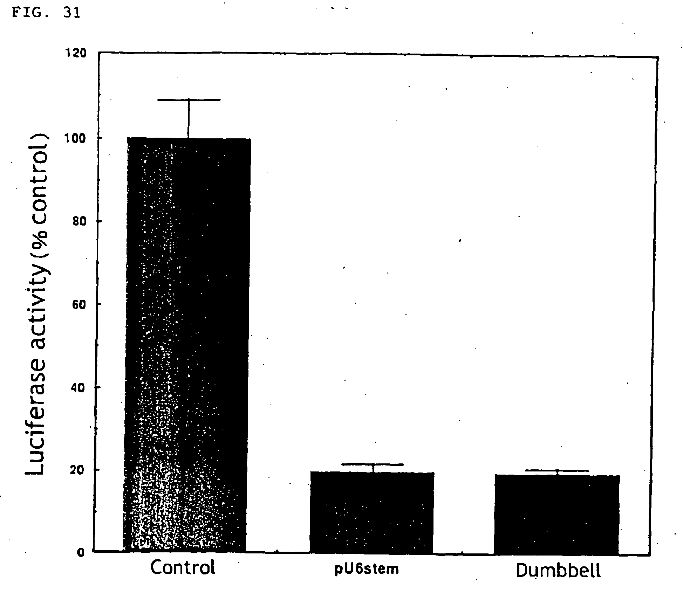

Fig. 31 is a graph representing the gene silencing effect of the siRNA expression dumbbell-shaped vector.

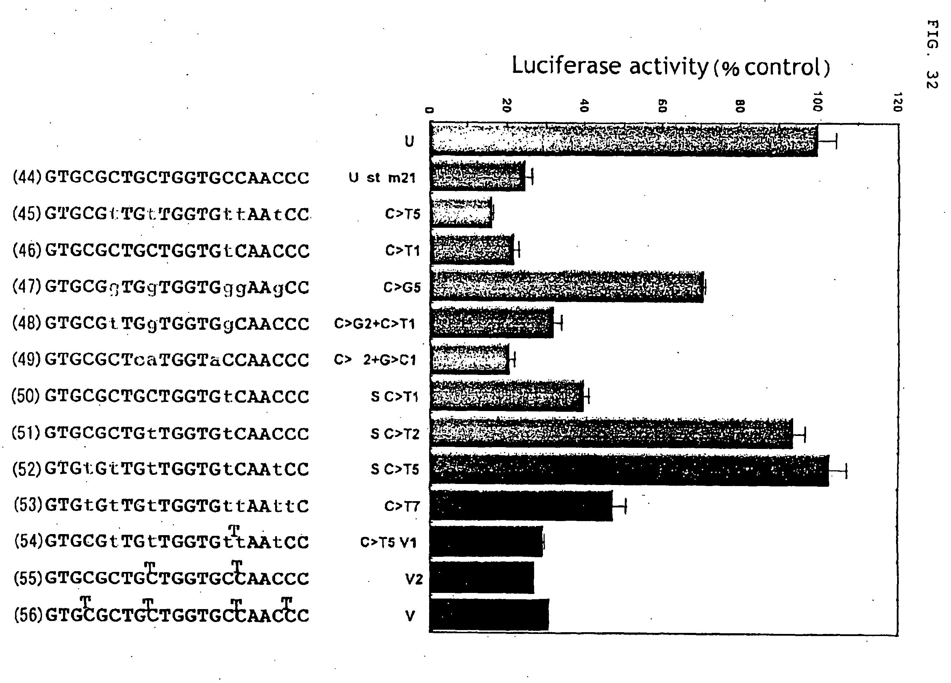

Fig. 32 is a graph representing the gene silencing effect of the siRNA expression system containing a mismatch or a bulge in the double-stranded RNA region of siRNAs. Numerals in parentheses at the top of each sequence represent SEQ ID NOs.

### Best Mode for Carrying out the Invention

### [Example 1] RNAi induction by using siRNA expression vector

Whether the siRNA expression vector can silence the target gene coding for the exogenous hygromycin/EGFP fusion protein was examined.

The Hygromycin/EGFP expression vector (pHygEGFP), and DsRed expression vector (pDsRed2) that is an internal control were purchased from Clontech. The siRNA expression vector was constructed using the plasmid pU6 carrying the human U6 promoter (Ohkawa, J. & Taira, K. Control of the functional activity of an antisense RNA by a tetracycline-responsive derivative of the human U6 snRNA promoter. Hum Gene Ther. 11, 577-585 (2000) ) . Fragments containing DNAs coding for portions of hygromycin/EGFP sense and antisense RNAs were synthesized with a DNA synthesizer, and subcloned immediately downstream of the U6 promoter in pU6. To insert these synthetic fragments downstream of the U6 promoter in pU6, a BspMI recognition site was provided downstream of the U6 promoter and another BspMI site was provided further downstream thereof in the vector used in subcloning. After cleavage with BspMI, the 4-nucleotide cohesive ends were formed. The vector capable of expressing sense RNA was constructed by inserting the synthetic sense code DNA whose end is complementary to these cohesive ends.

In a similar manner, DNA coding for antisense RNA (19 nucleotides) was also synthesized, and subcloned immediately downstream of the U6 promoter in pU6.

This antisense RNA expression cassette containing the U6 promoter was excised from the vector, inserted into the pU6 vector comprising the sense RNA expression cassette to construct the siRNA expression vector (pU6iHyg/EGFP). Herein, since it has been reported that, in the case of using the U6 promoter, four uridines (Us) are added to the 3'-end of the expressed mRNA, siRNA that is expressed by the siRNA expression vector and intracellularly formed has four nucleotide-overhangs at both 3'-ends. That is, this siRNA expression vector expresses a 23-nucleotide long siRNA having the duplex of 19 nucleotides and 4-nucleotide overhangs at both 3'-ends thereof (Fig. 1A).

Human HeLa S3 cells were co-transfected with the above-described pHygEGFP (1 µg), pDsRed2 (0.5 µg), and pU6iHyg/EGFP (1 µg) by the lipofection method (using Lipofectamine 2000). Forty-eight hours after the transfection, the cells were allowed to stand at 37°C, and observed under a confocal microscope. As a control experiment, similar operations were conducted using pU6 in place of the siRNA expression vector.

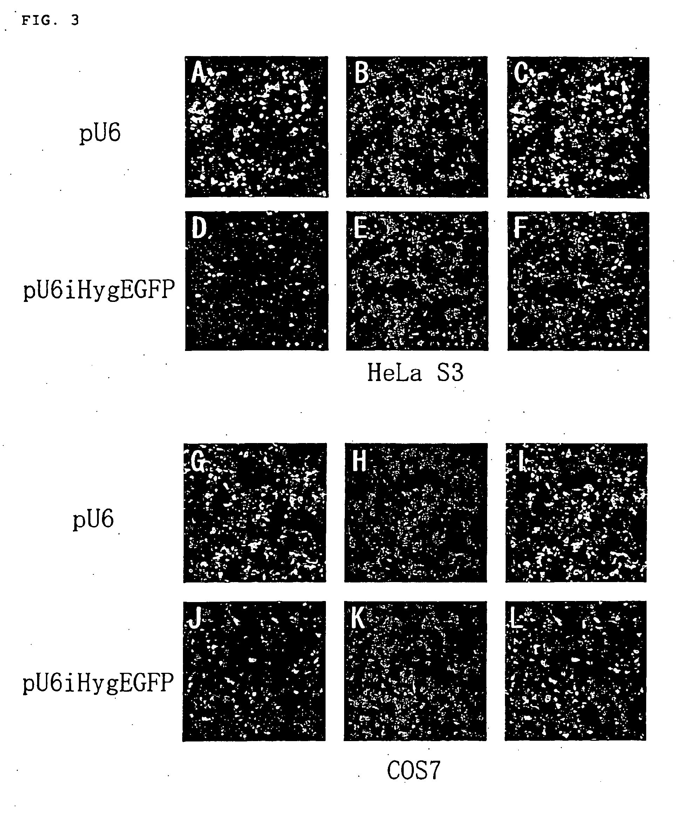

In Fig. 3, upper panels represent the results of control experiments using pU6, while lower panels show those obtained by introducing pU6iHyg/EGFP. As shown in the center column of Fig. 3, in cells transduced with the red fluorescence-emitting pDsRed as an internal control, no significant difference in the fluorescence intensity was observed between the control and pU6iHyg/EGFP-transduced groups, indicating no difference in the vector transfer efficiency between the two experimental groups, and also no non-specific gene silencing of gene expression by the siRNA expression vector. On the other hand, as shown in the left column of Fig. 3, cells emitting green fluorescence due to pHygEGFP were reduced in number and the fluorescence intensity in green fluorescent cells was also decreased in the pU6iHyg/EGFP-transduced group compared to the control group. Similarly, as shown in the right column of Fig. 3, even when red and green fluorescences were merged, green-fluorescent cells and yellow fluorescent cells due to the merging of red and green were decreased in number in the siRNA expression vector-transduced cells compared to the control. These results proved that introduction of the siRNA expression vector induces RNAi, leading to the target gene silencing. Furthermore, as a result of similar analysis conducted using mouse COS 7 cells, it was observed that the expression of pDsRed was not affected at all, but that of pHygEGFP was specifically silenced (not shown).

### [Example 2] Quantification of gene silencing activity of siRNA expression vector

In order to quantify RNAi effects, the gene expression silencing activity of siRNA directed against genes of firefly and sea pansy luciferases as the other reporter gene was analyzed as follows.

HeLa S3 and COS 7 cells were cultured in Dulbecco's modified Eagle's medium supplemented with 10% fetal bovine serum. The respective cultured cells (3 x 10⁴ cells/well) were placed in each well of 48-well plates. To conduct luciferase reporter analysis, the RSV-sea pansy luciferase expression vector (pRL-RSV)¹⁵ (30 ng), firefly luciferase expression vector pGL3 (Promega) (30 ng), and various amounts of siRNA expression vectors directed against firefly or sea pansy luciferase transcriptional product were co-transfected into cells in each well by the lipofection method using Lipofectamine 2000 (Life Technologies).

Results of luciferase analysis in HeLa S3 cells are shown in Fig. 4. Fig. 4A represents results obtained by the introduction of a fixed amount (300 ng) of the siRNA expression vector, indicating silencing of the corresponding target gene expression due to the siRNA introduction. Furthermore, in the luciferase activity analysis carried out with plasmids expressing only either sense or antisense RNA, both plasmids had no effect on the expression of firefly and sea pansy luciferases at all. Fig. 4B shows results obtained by introduction of varied amounts of siRNA. The siRNA expression vector directed against the firefly luciferase gene dose-dependently decreased the firefly luciferase activity without affecting the sea pansy luciferase activity. On the other hand, in cells transfected with the siRNA expression vector directed against sea pansy luciferase gene, sea pansy luciferase activity was dose-dependently reduced. These results clearly demonstrated that the siRNA expression system using the U6 promoter specifically and effectively silenced the target gene.

### [Example 3] Target site-dependent gene silencing

Next, it was examined whether siRNA expression vectors directed against different target sites in the same transcriptional product have different gene silencing effects or not. In this analysis, each of siRNA expression vectors directed against four different target sites on the firefly luciferase transcriptional product was co-transfected together with the firefly luciferase expression vector, and sea pansy luciferase expression vector as an internal control into HeLa S3 cells under similar conditions as in Example 2. Sequences of sense and antisense code DNAs in siRNA expression vectors directed against these four different target sites are set forth below:
firefly luciferase site 0 sense strand: 5'-GCTATGAAACGATATGGGC-3' (SEQ ID NO: 1);
site 0 antisense strand: 5'-GCCCATATCGTTTCATAGC-3' (SEQ ID NO: 2) ;
site A sense strand: 5'-GTTCGTCACATCTCATCTAC-3' (SEQ ID NO: 3) ;
site A antisense strand: 5'-GTAGATGAGATGTGACGAA-3' (SEQ ID NO: 4) ;
site B sense strand: 5'-GTGCGCTGCTGGTGCCAAC-3' (SEQ ID NO: 5) ;
site B antisense strand: 5'-GTTGGCACCAGCAGCGCAC-3' (SEQ ID NO: 6);
site C sense strand: 5'-ATGTACACGTTCGTCACAT-3' (SEQ ID NO: 7) ;
site C antisense strand: 5'-ATGTGACGAACGTGTACAT-3' (SEQ ID NO: 8);
control sea pansy luciferase
(sense strand with nucleotide overhang):
   5'-GTAGCGCGGTGTATTATAC-3' (SEQ ID NO: 9);
(antisense strand with nucleotide overhang):
   5'-GTATAATACACCGCGCTAC-3' (SEQ ID NO: 10).

As shown in Fig. 5A, the luciferase gene silencing activity varied depending on differences of target sites. That is, the siRNA expression vector directed against site B was highest in the gene silencing activity so that the firefly luciferase activity was reduced to 14% of the control. The siRNA expression vectors directed against sites A, C and D reduced the enzyme expression level to 44%, 38% and 36% of the control, respectively. In this case, the siRNA expression vector directed against site O showed no gene expression silencing activity similarly as the two control expression vectors, Hyg-U6siRNA and pU6.

It was also examined whether the above-described difference in the gene silencing activity depending on target sites is either derived solely from differences in target site or due to differences in the transcriptional efficiency of each siRNA. For this examination, the above-mentioned sense and antisense RNA oligonucleotides were synthesized respectively. Sequences of these siRNA oligonucleotides are as follows:
firefly luciferase site O sense strand:
   5'-GCUAUGAAACGAUAUGGGCUU-3' (SEQ ID NO: 11);
site O antisense strand: 5'-GCCCAUAUCGUUUCAUAGCUU-3' (SEQ ID NO: 12);
site A sense strand: 5'-GUUCGUCACAUCUCAUCUACUU-3' (SEQ ID NO: 13) ;
site A antisense strand: 5'-GUAGAUGAGAUGUGACGAAUU-3' (SEQ ID NO: 14);
site B sense strand: 5'-GUGCGCUGCUGGUGCCAACUU-3' (SEQ ID NO: 15) ;
site B antisense strand: 5'-GUUGGCACCAGCAGCGCACUU-3' (SEQ ID NO: 16) ;
site N sense strand: 5'-AUGUACACGUUCGUCACAUUU-3' (SEQ ID NO: 17) ;
site N antisense strand: 5'-AUGUGACGAACGUGUACAUUU-3' (SEQ ID NO: 18);
control sea pansy luciferase
(sense strand with nucleotide overhang):
   5'-GUAGCGCGGUGUAUUAUACUU-3' (SEQ ID NO: 19);
(antisense strand with nucleotide overhang):
   5'-GUAUAAUACACCGCGCUACUU-3' (SEQ ID NO: 20).

The above-described RNA oligonucleotides were synthesized using an RNA synthesizer Model 394 (Applied Biosystems). Synthetic RNAs were deprotected, and purified by denaturing acrylamide gel electrophoresis. After eluted from the gel, each RNA fraction was applied onto an NAP-10 column (Pharmacia) and eluted with water free from ribonucleases, for desalting. The resulting eluate was dried *in vacuo*, and re-suspended in annealing buffer (phosphate-buffered physiological saline (PBS) at pH 6.8, 2 mM MgCl₂) . Then, for annealing RNA oligonucleotides, 10 µM RNAs were prepared, incubated at 95°C for 1 min, then cooled to 70°C, and further slowly to 4°C over 2 hrs. The thus-obtained siRNA oligonucleotides were introduced into HeLa S3 cells similarly as described above to assay luciferase activity (Fig. 5B).

Luciferase gene silencing profiles by siRNA oligonucleotides directed against respective target sites showed a similar pattern to that obtained by the cases where siRNA expression vectors were introduced at 1 and 0.1 nM, except for a slight gene silencing activity expressed by siRNA oligonucleotide directed against site O. These results indicate that differences in the gene silencing activity are not caused by differences in the expression efficiency of each siRNA, but dependent on differences in target sites such as their secondary structures and the presence of RNA binding proteins.

### [Example 4] Effect of the length of 3' overhang of siRNA

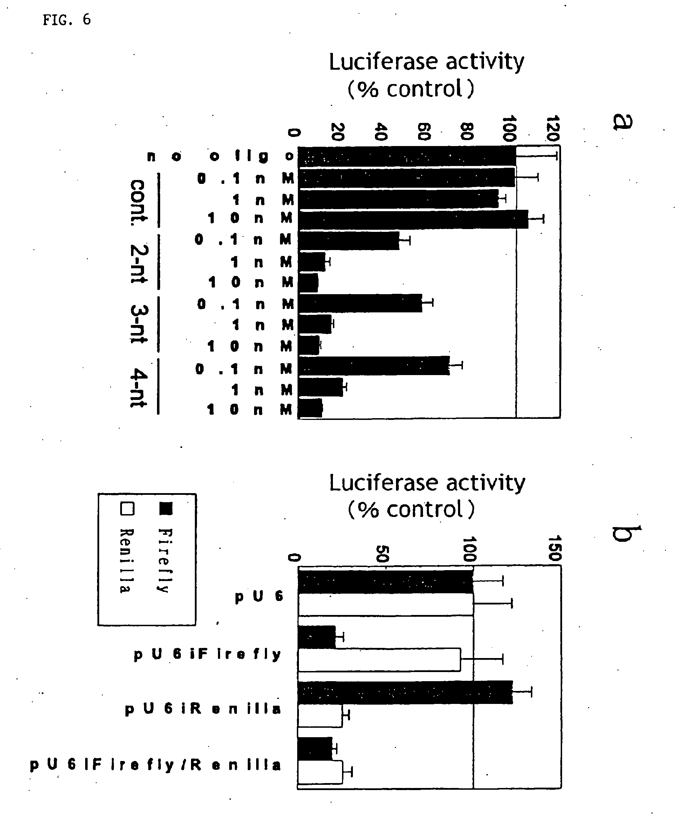

siRNAs produced by the U6 promoter have four uridine nucleotide overhangs at 3'-ends. On the other hand, Elbashir et al. reported that, in experiments *in vitro* using *Drosophila,* the gene silencing efficiency of siRNAs is reduced when 3' overhangs are longer than 2 to 3 nucleotides (Elbashir, S. M., Lendeckel, W. & Tuschl, T. RNA interference is mediated by 21- and 22-nucleotide RNAs. Genes Dev. 15, 188-200 (2001) ). Therefore, it was examined whether the four-nucleotide 3' overhang of the above-described siRNA affects the RNAi induction efficiency by siRNA or not. siRNA oligonucleotides directed against the same target site on the above-mentioned sea pansy luciferase transcriptional product with 3' overhangs whose number of uridine nucleotide is set to 2, 3 or 4 were prepared by chemical synthesis similarly as in the above-described Example 3. These siRNA oligonucleotides directed against the sea pansy luciferase transcriptional product, and firefly luciferase expression vector as an internal control were introduced at various concentrations into HeLa S3 cells by the lipofection method to assay luciferase activity (Fig. 6A).

Expression of sea pansy luciferase was not silenced with the above-described internal control alone, but dose-dependently suppressed in a group of cells transduced with the above-mentioned siRNA oligonucleotides. Furthermore, no significant difference in the gene silencing activity due to the 3' overhang varying from 2 to 4-nucleotide long was observed, thereby revealing that siRNA with 4-nucleotide 3' overhang produced from the U6 promoter is also capable of silencing the target gene expression as effectively as siRNAs with 2-, or 3-nucleotide overhang.

### [Example 5] Simultaneous silencing of a plurality of genes

It was examined whether different target genes can be simultaneously silenced by siRNA when two different genes including the target genes are expressed at the same time. A plasmid containing two siRNA expression cassettes directed against firefly and sea pansy luciferases was constructed, and co-transfected into cells.

Firefly luciferase expression vector (30 ng), sea pansy luciferase expression vector (30 ng), a vector (300 ng) expressing siRNAs directed against both luciferase transcriptional products were co-transfected together with a vector (100 ng) expressing β-galactosidase as an internal control into HeLa S3 cells. As the control, similar experiments were carried out using vectors expressing siRNA directed against either one of firefly and sea pansy luciferase transcriptional products.

As shown in Fig. 6B, transfection of the siRNAs expression vector (U6i-Firefly/Renilla) directed against both luciferases simultaneously silenced the expression of firefly and sea pansy luciferases to the same levels as that when siRNA expression vector directed against either one of luciferases (U6i-Renilla or U6i-Firefly) was introduced.

As described above, it was demonstrated that, by arranging a plurality of siRNA expression cassettes in the same plasmid to simultaneously express these multiple siRNAs, it is possible to silence corresponding target genes without generating interference among respective promoters.

### [Example 6] Endogenous gene silencing

All of the above-mentioned Examples related to the silencing of exogenous gene introduced into cells. In this experiment, whether siRNA expression vector is capable of silencing the endogenous gene expression was examined.

The endogenous gene coding for β-catenin was selected as a target. This β-catenin is a membrane-tethered cytoplasmic protein, and is known as a factor associated with cadherins in intercellular adhesion and also as an important oncogene (Peifer, M. & Polakis, P. Wnt signaling in oncogenesis and embryogenesis - a look outside the nucleus. Science 287, 1606-1609 (2000) ).

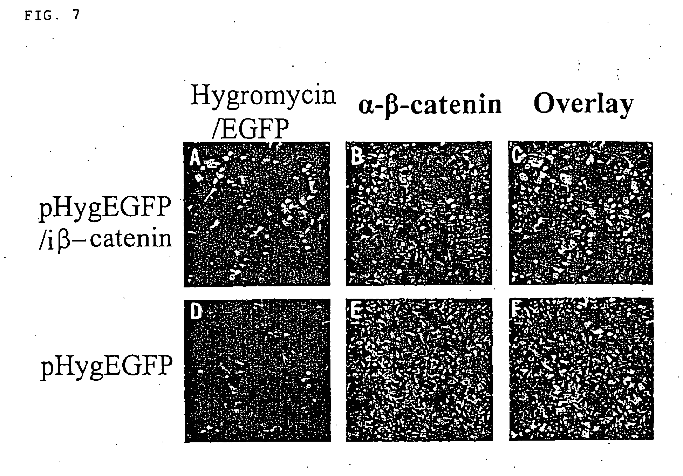

EGFP expression plasmid containing the siRNA expression cassette directed against β-catenin (pEGFP/ibeta-catenin) was introduced into SW480 cells expressing β-catenin. As a control, EGFP expression plasmid containing no siRNA expression cassette directed against β-catenin (pEGFP) was similarly introduced into the cells at 60% confluency. These plasmids were introduced into the cells mounted on slide glass using reagents such as Effectene (Qiagen) or Fugene 6 (Roche Molecular Biochemicals). Forty eight hours after transduction, cells were fixed in PBS containing 4% paraformaldehyde for 20 min, permeabilized in 0.1% Triton X100, then stained using the anti-β-catenin antibody (UBI) and Cy3-labeled secondary antibody. Cellular fluorescence after staining was analyzed using a confocal microscope (Fig. 7).

It was demonstrated that, in green fluorescent cells holding pEGFP/ibeta-catenin, the β-catenin expression level was substantially low as compared with the cells transduced with no plasmid. Furthermore, no difference in the β-catenin expression level was observed in green fluorescent cells transduced with pEGFP and cells transduced with no plasmid.

### [Example 7] Comparison of gene silencing effects between the tandem and stem-loop siRNA expression vectors

Gene silencing effects of an siRNA expression vector (pU6tandem19) in which DNAs coding for sense and antisense RNAs are arranged in tandem and an siRNA expression vector (pU6stem19) capable of expressing the stem loop RNA molecule, were examined. The siRNAs expressed from the above-described respective vectors are referred to as tandem siRNA and stem-loop siRNA. The sequence of the stem-loop siRNA transcribed in the pU6stem19 is 5'-GTGCGCTGCTGGTGCCAACgugugcuguccGTTGGCACCAGCAGCGCAC-3' (SEQ ID NO: 21). The gene silencing activity was quantified by the luciferase analysis described in the above-described Examples.

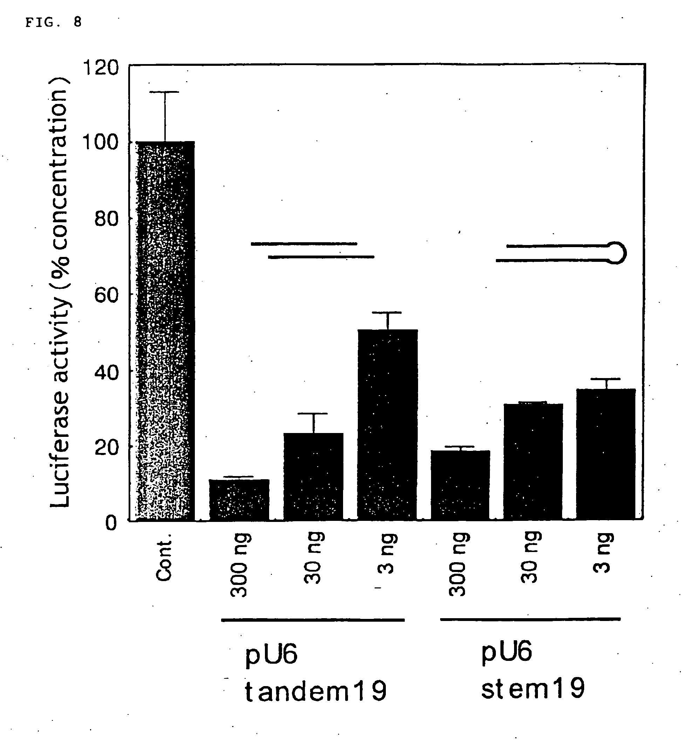

Results are shown in Fig. 8. Both tandem and stem-loop siRNAs concentration-dependently reduced the luciferase activity. These results revealed that both the tandem and stem-loop siRNA expression systems effectively suppress the expression of the target gene.

### [Example 8] Gene silencing effects of various siRNA expression vectors

Gene silencing effects of siRNA expression vectors containing various promoters were examined. Luciferase analysis was performed under similar conditions as in the above-described Examples. The following siRNA expression vectors were used.
· vector (pU6tandem19) expressing the tandem siRNA by human U6 promoter,
· vector (p5Standem19) expressing the tandem siRNA by human 5S rRNA promoter,
· vector (pHltandeml9) expressing the tandem siRNA by human H1 promoter, and
· vector (pH1stem19) expressing the stein-loop siRNA by human H1 promoter.

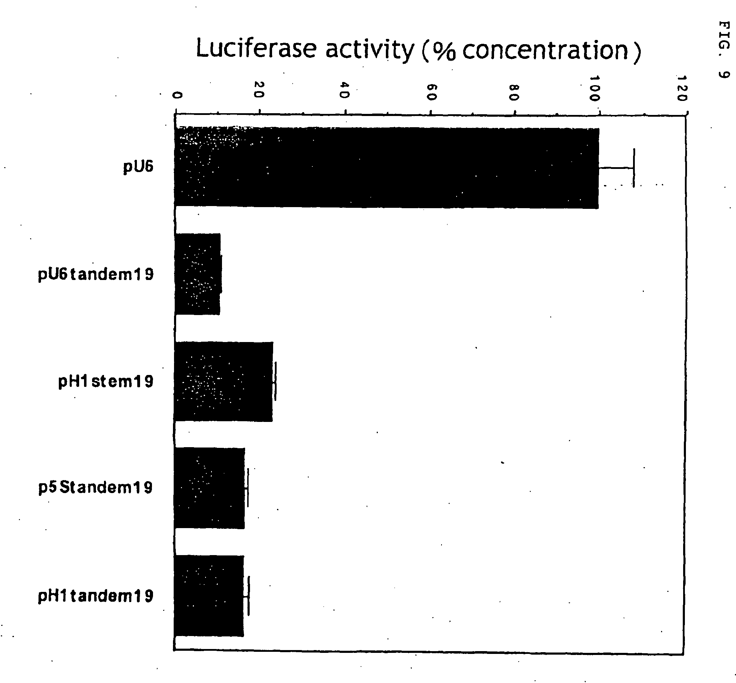

As shown in Fig. 9, various expression vectors used herein showed the luciferase suppressing activity, indicating that promoters usable in the siRNA expression vector are not limited to specific promoters, and that various promoters such as human U6 promoter, human 5S rRNA promoter, and human H1 promoter can be utilized.

### [Example 9] Gene silencing effects of siRNA expression vector containing CMV promoter or tRNA promoter

Gene silencing effects of the siRNA expression vector containing cytomegalovirus-derived promoter (CMV promoter) or tRNA promoter, and trimming ribozyme (pCMV-TRz and ptRNA-TRz, respectively), was examined. The transcript from tRNA promoter has a tRNA molecule added to the 5' end. The excessive RNA molecule at the 3' end which is unnecessary for the siRNA formation is cut off by the action of the trimming ribozyme.

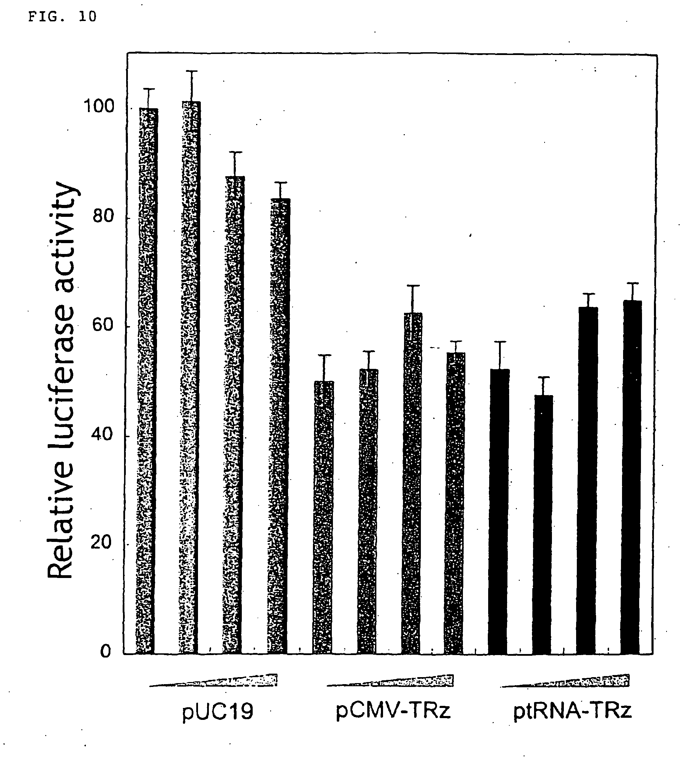

As shown in Fig. 10, siRNA expression vectors containing either CMV promoter or tRNA promoter both decreased the luciferase activity, indicating that, as a promoter in the siRNA expression vector of this invention, CMV promoter or tRNA promoter can be preferably used, and that even the transcript of the expression vector, which binds to a molecule such as tRNA at the 5' end, has the target gene silencing effect.

### [Example 10] RNAi induction by double-stranded siRNA containing mismatch or bulge

Effects of the presence of mismatch or bulge in the double-stranded siRNA on RNAi were examined. Luciferase analysis was performed under similar conditions to those in the above-described Examples. The following RNA sequences were used in experiments.
control RNA sequence:
   5'-GUGCGCUGCUGGUGCCAACCCgugugcuguccGGGUUGGCACCAGCAGCGCAC-3' (SEQ ID NO: 22)
· RNA sequence containing a mismatch:
   5'-GUGCGCUGuUGGUGuCAACCCgugugcuguccGGGUUGGCACCAGCAGCGCAC-3' (SEQ ID NO: 23)
· RNA sequence containing a bulge:
   5'-GUGCGCUGCUGGUGCuCAACCCgugugcuguccGGGUUGGCACCAGCAGCGCAC-3' (SEQ ID NO: 24)

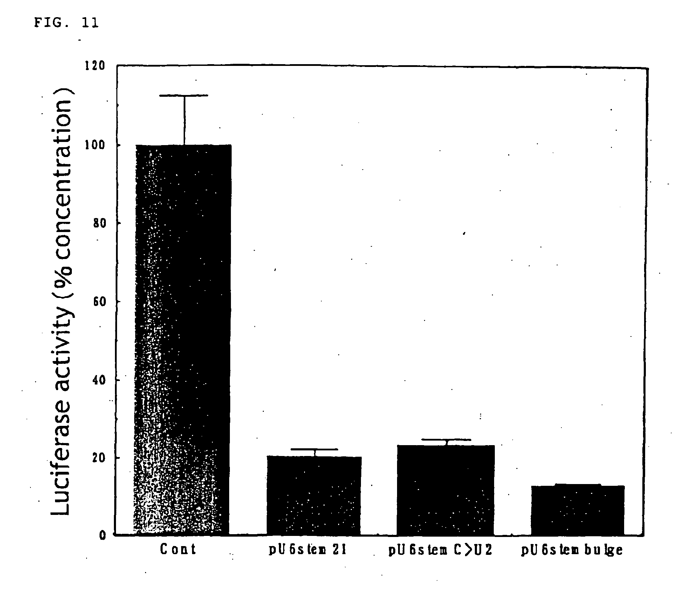

As shown in Fig. 11, various expression vectors used herein showed the luciferase suppression activity. The presence or absence of mismatch or bulge in the double-stranded siRNA produced no significant difference in the gene silencing effect.

Furthermore, RNAi effects induced by various siRNAs containing a mismatch or a bulge were examined. DNA sequences coding for one of the strands of siRNAs, and RNAi effect (luciferase activity) induced by the sequences are shown in Fig. 32.

These results demonstrated that even siRNA containing a mismatch or a bulge in its double strand is capable of effectively suppressing the expression of the target gene. That is, each strand constituting the double strand of siRNA is not necessarily completely complementary to each other.

### [Example 11] RNAi induction by siRNA expression vector having a tetracycline-inducible promoter

A system (Tet-ON system) capable of controlling the transcriptional activity from RNA promoter by tetracycline is known (Ohkawa, J. & Taira, K. Control of the functional activity of an antisense RNA by a tetracycline-responsive derivative of the human U6 snRNA promoter. Hum Gene Ther. 11, 577-585 (2000)). The tetracycline operator sequence of tetracycline-resistance transposon has been inserted into human U6 promoter used in this system (Fig. 12). Binding of the tetracycline repressor protein to this sequence results in the suppression of promoter activity. Expression vectors whose transcription is controlled by this promoter is in a state that the transcriptional activity is suppressed in cells (e.g. HeLa cell) expressing tetracycline repressor protein. This may be because tetracycline repressor protein in cells binds to human U6 promoter so as to suppress the transcriptional activity. When tetracycline (or tetracycline derivative) is added to the cells, it binds to the tetracycline repressor protein to release the repressor protein from U6 promoter, leading to the transcriptional activation.

The present inventors constructed an siRNA expression vector having human U6 promoter into which tetracycline operator sequence of tetracycline resistant transposon has been inserted, and examined the gene silencing effect of siRNA expression vector using the TetON system. Luciferase analysis was conducted under the similar conditions to the above-described Examples.

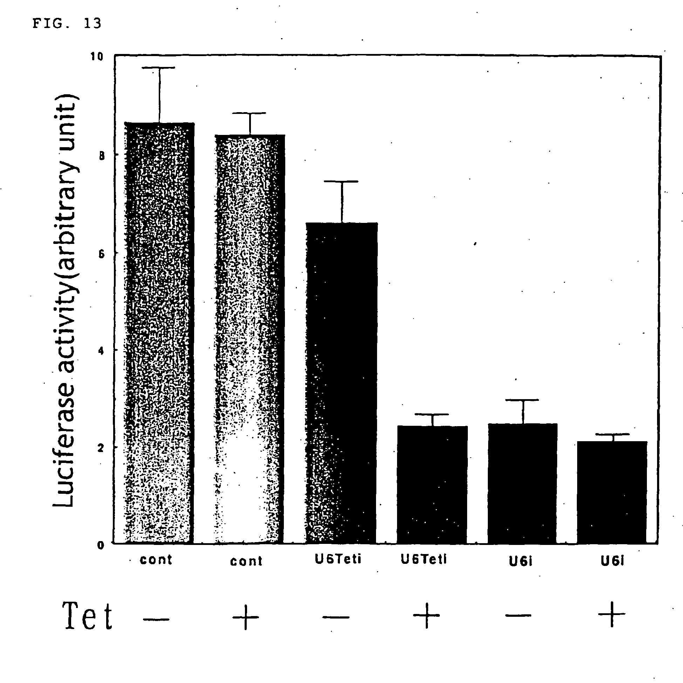

As shown in Fig. 13, the siRNA expression vector containing human U6 promoter into which the tetracycline operator sequence of tetracycline-resistant transposon has been inserted, reduced the luciferase activity upon addition of tetracycline. On the other hand, the siRNA expression vector containing no tetracycline operator sequence reduced the luciferase activity regardless of addition or no addition of tetracycline. These results demonstrated that, as a promoter that induces siRNA expression, the above-described U6 promoter inducible by tetracycline can be preferably used.

### [Example 12] Production of siRNA by RNA self-processing

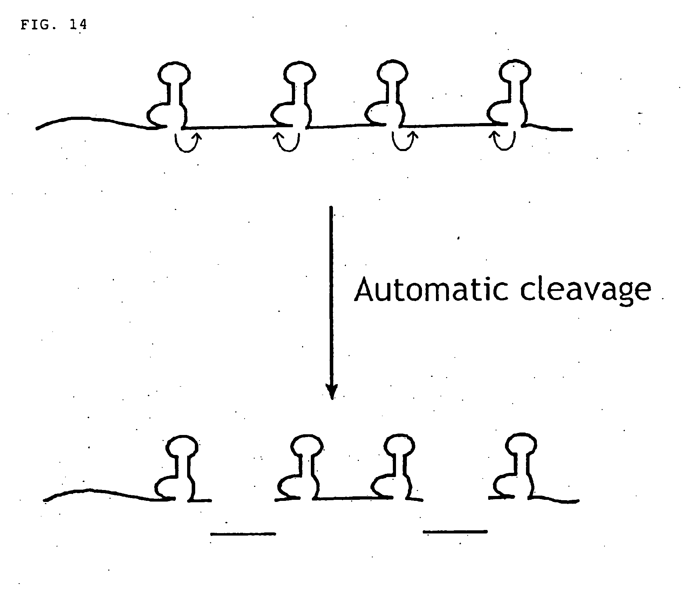

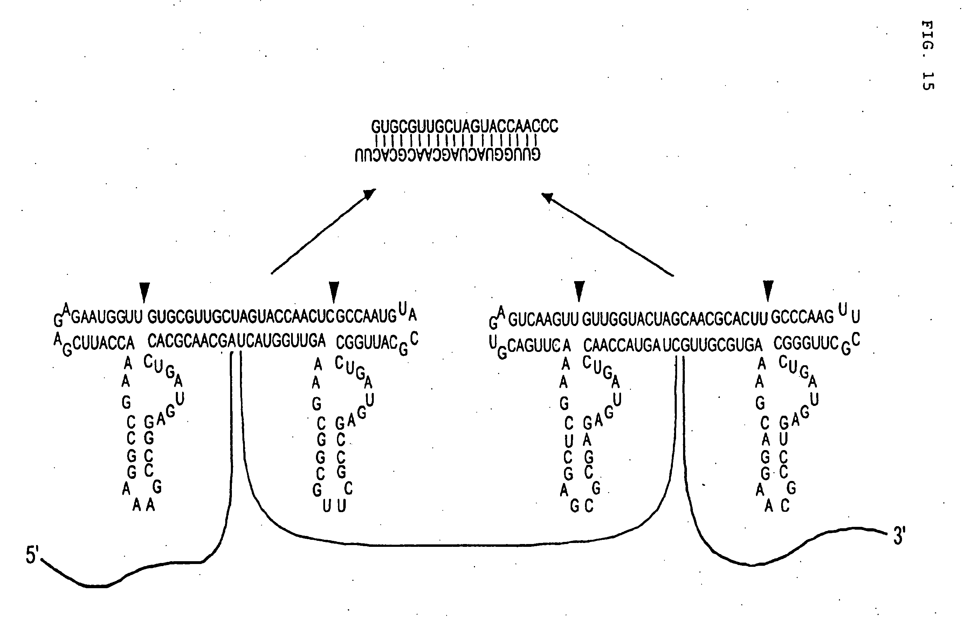

The use of pol II promoter in the siRNA expression vector results in the transcription of somewhat long RNA. Therefore, in the case of using pol II promoter, it is necessary to cleave this RNA, for example, by self-processing to produce antisense RNA or sense RNA. Using an RNA producing unit (Fig. 2) containing antisense code DNA or sense code DNA, a region coding for RNA sequence to be recognized by ribozyme (recognition sequence coding region) at their 5' and 3' ends, and, flanking outside of this recognition sequence coding region, a region coding for the 5' and 3' end cleaving ribozyme that cleaves the recognition sequence coding region, whether the self-processing actually occurs or not was examined. Figs. 14 and 15 are diagrams representing the RNA self-processing. Transcripts from the above-described unit were subj ected to gel electrophoresis.

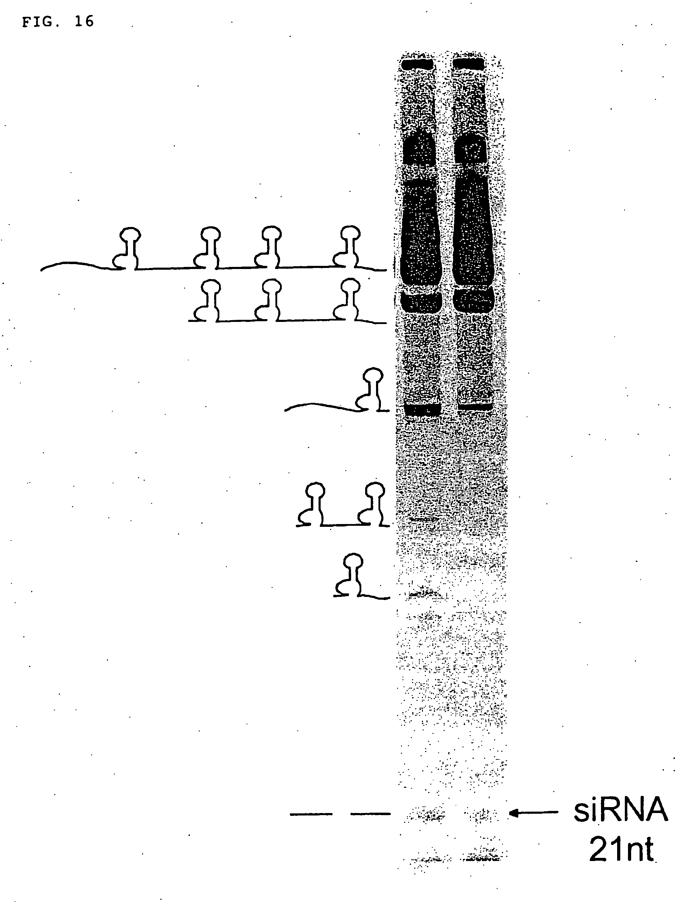

Results of gel electrophoresis are shown in Fig. 16. On the left side of electrophoresed bands in Fig. 16, putative structures of RNA molecules corresponding to respective bands are schematically shown. Several bands corresponding to RNAs of various lengths which were thought to be produced by RNA self-processing, were observed.

A 21 nt-long siRNA band was also observed. These results indicated that siRNAs can efficiently be produced due to the RNA self-processing effect using such an RNA producing unit as shown in Fig. 2.

### [Example 13] Preparation of siRNA library expression system targeting EGFP mRNA

An siRNA library expression system targeting random sites of EGFP mRNA was prepared. Outlines of the preparation are shown in Figs. 19 through 23.

### (a) Preparation of approximately 20 to 25 bp EGFP cDNA fragments

"Approximately 20 to 25 bp long random EGFP cDNA fragments having the dephosphorylated blunt ends" that are the starting materials for the preparation of the siRNA expression library shown in Fig. 19-① were prepared as described below. The outline of its preparation is shown in Fig. 24.

The EGFP coding region was amplified from pEGFP-N1 by PCR, and approximately 20 to 25 bp long random EGFP cDNA fragments were obtained by the DNase I treatment of the amplification products. For a large-scale preparation of the fragments, the fragments thus obtained was blunted with Klenow Fragment, and subcloned into the pSwaI vector that have been constructed by modifying pUC18. This vector contains the recognition site of the restriction enzyme BseRI in the upstream and downstream regions of the cloning site (SwaI recognition site) so as to excise DNA insert by BseRI.

The approximately 20 to 25 bp long EGFP cDNA fragments thus subcloned were excised with BseRI. The two nucleotide excessive cohesive ends formed after the BseRI cleavage were blunted using T4 DNA Polymerase, and then, both ends were dephosphorylated by the CIAP treatment. The above-described procedures yielded a large quantity of the approximately 20 to 25 bp long random EGFP cDNA fragments having dephosphorylated blunt ends.

### (b) Synthesis of DNA fragment having sense code DNA and antisense code DNA of siRNA targeting EGFP mRNA

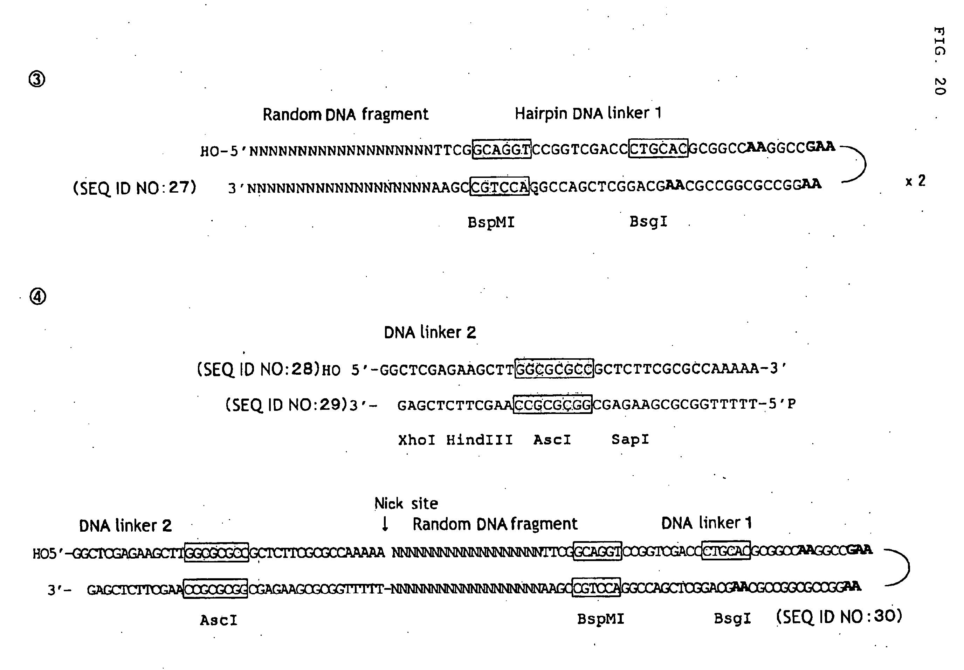

To the both ends of the "approximately 20 to 25 bp long EGFP cDNA fragment having dephosphorylated blunt ends" prepared in (a), the 5'-phosphorylated hairpin linker 1 was ligated (Fig. 19-②). This hairpin liker contains recognition sites of type II restriction enzymes, BspMI and BsgI, so that it can be cleaved at the linkage site or its vicinities. The strand displacement reaction was carried out by allowing Bst DNA Polymerase to react to the Nick site present in the linkage site of the ligation product of this approximately 20 to 25 bp long cDNA fragment with the hairpin linker. Thus, a DNA product, in which the approximately 20 to 25 bp long EGFP cDNA fragment was ligated with the hairpin linker at 1:1 ratio was synthesized (Fig. 20-③) Next, to the blunt end side (EGFP cDNA fragment side) of this product, the 5'-end phosphorylated DNA linker 2 was ligated (Fig. 20-④) AAAAA/TTTTT sequence signaling the termination of transcription from Pol III promoter is present at the one end of this DNA linker, while the AscI recognition site is present at the other end. Only the AAAAA/TTTTT side is 5'-phosphorylated.

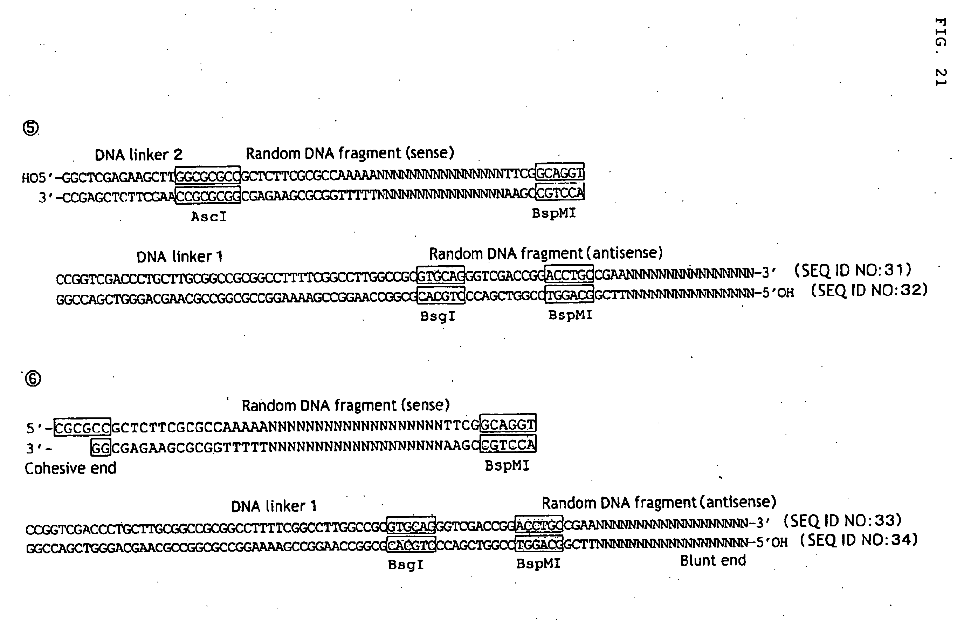

This ligation product of EGFP cDNA, linker 1, and linker 2 was reacted again with Bst DNA Polymerase to perform the strand displacement reaction from the Nick site at the linkage site (Fig. 21-⑤). Thus, the DNA fragment, in which DNAs coding for sense and antisense RNAs of siRNA targeting EGFP mRNA were arranged in tandem, was synthesized. In this fragment, the recognition sites of type II restriction enzymes, BspM1 and BsgI, are present between the sense code DNA and the antisense code DNA.

### (c) Cloning of DNA fragment having DNAs coding for sense and antisense of siRNA targeting EGFP mRNA

The DNA fragment synthesized in (b) having DNAs coding for sense and antisense of siRNA targeting EGFP mRNA was cleaved with AscI to prepare a DNA fragment having cohesive and blunt ends (Fig. 21-⑥). For cloning this fragment, a cloning vector containing human U6 promoter was constructed (Fig. 25). Recognition sites of restriction enzymes BspMI, BseRI, and AscI are present downstream of this promoter. After the cleavage immediately downstream of the promoter with BspMI, the cleavage site was blunted with Klenow, and the product was cleaved with AscI to prepare a cloning vector having cohesive and blunt ends. This vector was ligated to the DNA fragment with blunt and cohesive ends having DNAs coding for sense and antisense of siRNA targeting EGFP mRNA, and then cloned (Anti-EGFP U6 siRNA expression pre-library) (Fig. 22-⑦).

A cloning vector containing human tRNA-val promoter was also constructed (Fig. 25). Recognition sites of restriction enzymes BspMI, BseRI, and AscI are similarly present downstream of human tRNA-val promoter. This cloning vector can also be prepared by cleavage with BseRI instead of BspMI. This vector was cleaved immediately downstream of the promoter with BseRI; the cleaved site was blunted with T4 DNA polymerase, then the product was cleaved with AscI and similarly cloned (Anti-EGFP tRNA siRNA pre-library).

The siRNA expression pre-library plasmid DNA was cleaved at BspMI site between the siRNA sense and antisense code DNAs (Fig. 22-⑧). The cleavage fragment was blunted with Klenow and self-ligated to construct the Anti-EGFP siRNA expression library in which promoter, antisense DNA, sense DNA and TTTTT were linked in tandem (Fig. 22-⑨). Sequence analysis confirmed that a desired product was produced. Examples of the sequence of DNAs coding for siRNA in the Anti-EGFP U6 siRNA expression library are shown in Table 1.

**Table 1**

| | | | |
|---|---|---|---|
| 5'-U6 promoter- (antisense code DNA) (no loop) (sense code DNA) TTTTT AscI-3' | | | |

| Clone No | Antisense code DNA | Loop | Sense code DNA |
|---|---|---|---|
| 1 | GFP 24 | None | GFP 24 |
| | CCCGTGCCCTGGCCCACCCTCGTG | | CACGAGGGTGGGCCAGGGCACGGG |
| | (SEQ ID NO: 39) | | (SEQ ID NO: 40) |
| 2 | GFP 24 | None | GFP 24 |
| | ACCAGGATGGGCACCACCCCGGTG | | CACCGGGGTGGTGCCCATCCTGGT |
| | (SEQ ID NO: 41) | | (SEQ ID NO: 42) |

In Table 1, the orientation of the antisense code and sense code might be the reverse of that in EGFP mRNA in some cases, but this does not affect RNAi induction.

An siRNA expression library having a Loop sequence of siRNA, TTCG, between antisense and sense DNAs was constructed by cleaving the pre-library plasmid DNA first with BsgI and then with BspMI (Fig. 23-⑧-2) , blunting the cleavage site with T4 DNA polymerase, and then performing self-ligation. It is possible to freely alter the loop sequence between antisense and sense code DNAs and add fragments of promoters, selection markers, etc., by altering the sequence of hairpin linker 1, and the type and site of restriction enzymes.

### [Example 14] Evaluation of siRNA library expression system targeting EGFP mRNA

The above-described Anti-EGFP U6 siRNA expression library (1 µg), and pEGFP-N1 (0.01 µg) were co-transfected into human HeLa S3 cells by the lipofection method (Lipofectamine 2000). The cells were allowed to stand at 37°C for 48 h and then observed under a confocal microscope. As a control experiment, similar operations were conducted using pUC18 (1 µg) in place of the siRNA library expression system.

As shown in Fig. 26, cells emitting green fluorescence due to pEGFP-N1 decreased in number in the group transduced with the Anti-EGFP U6 siRNA library expression system compared to the control group (pUC18-introduced group). Moreover, results of FACS analysis revealed the decrease in the cellular fluorescence intensity (Fig. 27). These results indicated that the introduction of the anti-EGFP U6 siRNA library expression system into cells induced RNAi so as to silence the target gene.

Thus, clones capable of inducing RNAi and silencing the target gene would be present in the anti-EGFP U6 siRNA library expression system.

Similar results were obtained in the Anti-EGFP tRNA siRNA library expression system (Figs. 26 and 27).

### [Example 15] Gene silencing effects of siRNA expression adenovirus vector and HIV vector

As a marker gene for evaluation, a luciferase gene (pGL3-Control: Promega) was used. siRNA was expressed in tandem using human U6 promoter. The target sequence was site B strand: 5'-GTGCGCTGCTGGTGCCAAC-3' (SEQ ID NO: 43). An adenovirus vector was prepared according to the method of Mizuguchi et al. (Nippon Rinsho, 58, 1544-1553 (2000)).

### 1) Incorporation of RNAi expression cassette into shuttle plasmid

Although there were three HincII sites in the sequence of PShuttle available from Clontech, it was confirmed, as a result of sequencing, that only one HincII site was present between I-CeuI and PI-SceI sites.

After the expression plasmid (pU6i-FGLB) was cleaved with HindIII, the ends were blunted by the Klenow treatment, and then cleaved with EcoRI. The expression cassette thus prepared (approximately 600 bp) was incorporated into the shuttle vector (pShuttle), which had been treated with EcoRI and HincII, to construct pU6i-FGLB/Shuttle.

### 2) Incorporation of expression cassette from pShuttle into Ad vector plasmid, and preparation of Ad vector

The RNAi expression cassette was incorporated into Ad vector plasmid (pAdHM15-RGD) having RGD fiber according to the method of Mizuguchi et al. to construct pU6-FGLB/RGD.

As a control, after pAdHMlS-RGD and pU6-FGLB/RGD with no insert were digested with PacI, lipofection was performed using TransIT293 (TaKaRa). From cells in which CPE was observed, Ad vector was prepared according to the method of Mizuguchi et al.

Ad vector prepared by the cesium chloride ultracentrifugation method was purified by dialysis against PBS(-) containing 1% BSA overnight. Titer of the purified Ad vector was measured using an Adeno-X Rapid Titer Kit (Clontech). Measured titers of respective Ad vectors were as follows:

| | |
|---|---|
| RGD/Ad (control): | 6.76×10^10 ifu/ml |
| U6-FGLB/Ad: | 5.27×10^10 ifu/ml |

### 3) Preparation of HeLa-S3 cell

HeLa-S3 cells were prepared to a density of 5 × 10⁵ cells/ml, and seeded 1 ml/well each in a 6-well plate. Then, each Ad vector was added to each well at a Moi of 1, 10, 20, 50, and 100. Twenty-four hours later, the medium (1.5 ml) was added prior to lipofection.

### 4) Lipofection of luciferase plasmid

Twenty-four hours after the transduction of Ad, the cells were lipofected with the luciferase expression plasmid in the following composition per well.

pGL3-Control (0.02 µg), pRL-Tk (0.1 µg), and pUC19 (1.0 µg) were added to Opti-MEM (250 µl) placed in Tube A. LipofectAmine 2000 (5 µl; Invitrogen) was added to Opti-MEM (250 µl) placed in Tube B, and the mixture was allowed to stand at room temperature for 5 min. The whole quantity of Tube B was added to Tube A, and the mixture was thoroughly mixed. After the resulting mixture was allowed to stand at room temperature for 20 min, the whole quantity was added to each well, and incubated at 37°C for 48 h.

### 5) Luciferase assay

Luciferase assay was carried out using a Dual-Luciferase Reporter Assay System (Promega).

After culturing for 48 h following the lipofection, each well in the plate was washed once with PBS (-) (500 µl). After the removal of PBS(-), 1 x PLB (500 µl) was added to each well, and the plate was occasionally shaken at room temperature for 15 min so as to lyse the cells. Cell lysate was transferred into a 1.5-ml tube, centrifuged at 14000 rpm for 1 min, and the supernatant was transferred into a fresh 1.5-ml tube (PLB lysate).

Luciferase activity was measured using an AutoLumatPLUS LB953 (Berthold). Using PLB lysate (10 µl), luminescence of FireFly Luciferase and Renilla Luciferase was measured for 10 s. Relative luciferase silencing effects of the respective RNAi expression Ads were expressed based on the RLU values of FireFly Luciferase/Renilla Luciferase, taking the value of cells transduced with the control RGD/Ad as 100% (Fig. 29).

Similarly, luciferase suppressing effects of RNAi by expression HIV vectors were observed as shown in Fig. 30.

siRNA expression HIV vectors were prepared in the substantially same manner as for the siRNA expression Adenovirus vector by inserting the siRNA expression cassette targeting FireFly luciferase into an HIV shuttle vector. In this assay, the target sequence was siteB strand: 5'-GTGCGCTGCTGGTGCCAAC-3' (SEQ ID NO: 43), U6 promoter was used, and, RNAs to be expressed have the stem-loop including 5'-GUGCGCUGCUGGUGCCAACCCgugugcuguccGGGUUGGCACCAGCAGCGCAC (SEQ ID NO: 22),
5'-GUGCGCUGuUGGUGuCAACCCgugugcuguccGGGUUGGCACCAGCAGCGCAC-3' (SEQ ID NO: 23), and
5'-GUGCGuUGuUGGUGuuAAuCCgugugcuguccGGGUUGGCACCAGCAGCGCAC-3' (SEQ ID NO: 57). After the siRNA expression shuttle plasmid was introduced into 293T cells, viral particles were harvested by the standard method, concentrated, and transfected into 293T cells at a moi of 5 to 8. Then, RNAi effects were examined in terms of luciferase activity similarly as in the case of the Adenovirus vectors.

### [Example 16] Gene silencing effects of siRNA expression dumbbell-shaped vector

Gene silencing effects of siRNA expression dumbbell-shaped vectors were examined. The following siRNA expression vectors were used, and luciferase analysis was performed under similar conditions as in the above-described Examples.
· vector expressing stem-loop siRNA using human U6 promoter (pU6stem), and
dumbbell-shaped vector expressing siRNA (Dumbbell)

As shown in Fig. 31, the luciferase silencing effect was observed in the siRNA expression dumbbell-shaped vector, demonstrating that the siRNA expression system maintained in the dumbbell-shaped vector exhibited an efficient gene silencing effect.

### Industrial Applicability

As described above, the use of the intracellular siRNA expression system enabled to silence the functional gene expression. Furthermore, as a result of introducing a single vector that has been transformed to maintain siRNA expression systems for a plurality of target genes into cell, the expression of multiple target genes could also be silenced. By using such an intracellular siRNA expression system, siRNA is supplied within the cell so as to enable the stable and long-term siRNA expression, that is, target gene silencing as well. In addition, by using viral vectors or the like, transfer efficiency of siRNA expression system into cells can be improved so as to allow the RNAi induction in mammalian cells without fail. Therefore, the present system is able to contribute to the gene therapy and production of knock-down animals depending on RNAi.

Furthermore, in order to allow the present system to be applied to a method of searching for functional genes, the siRNA library expression system and its assembly have been provided. The use of these systems and the like can make the searching for functional genes so simple and efficient that the present systems including siRNA library expression system can contribute to the accelerated elucidation of functional genes.

## Claims

1. An intracellular siRNA expression system,
wherein said siRNA comprises a double strand region in which an antisense RNA strand and a sense RNA strand complementary to the antisense RNA pair up, and is directed against a region of a target gene mRNA in a mammalian cell, and
wherein said antisense and sense RNA strands are produced from a vector comprising a DNA encoding said antisense RNA strand and a DNA encoding said sense RNA strand, and one or more pol III promoters capable of producing said antisense and sense RNA strands,
wherein said vector comprises any one of the following (a) to (c):
a) a stem-loop siRNA producing unit comprising a DNA encoding the antisense RNA strand and a DNA encoding the sense RNA strand complementary to the antisense RNA strand which are linked in the opposite direction via a linker, and a pol III promoter capable of producing said stem-loop siRNA at either side of said unit, wherein the double-stranded RNA region of the siRNA contains 1 to 7 mismatches and/or 1 to 7 bulges, and wherein one of nucleotides in the mismatch is guanine, and the other is uracil,
b) a double-stranded DNA region in which a DNA encoding the antisense RNA strand and a DNA encoding the sense RNA strand complementary to the antisense RNA strand pair up, and two pol III promoters facing to each other with said double-stranded DNA region in between which are capable of producing said antisense and sense RNA strands, or
c) a DNA encoding the antisense RNA strand, a DNA encoding the sense RNA strand complementary to the antisense RNA strand, and pol III promoters at the 5' ends of the antisense and sense code DNAs which are capable of producing said antisense and sense RNA strands.

2. The siRNA expression system according to claim 1, wherein the double strand region of the siRNA expressed by the system is 15 to 49 bp long.

3. The siRNA expression system according to claim 1, wherein the double strand region of the siRNA expressed by the system is 15 to 35 bp long.

4. The siRNA expression system according to claim 1, wherein the double strand region of the siRNA expressed by the system is 15 to 30 bp long.

5. The siRNA expression system according to any one of claims 1 to 4, wherein said pol III promoter is U6 promoter.

6. The siRNA expression system according to any one of claims 1 to 5, wherein said promoter is an inducible promoter.

7. The siRNA expression system according to any one of claims 1 to 6, wherein said system comprises IoxP sequences in the form of any one of the following (a) to (c) so that the expression can be controlled:
(a) the promoter comprises distal sequence element (DSE) and proximal sequence element (PSE) with a space therebetween, and in the space two IoxP sequences, one in the vicinity of DSE and the other in the vicinity of PSE;
(b) the promoter comprises DSE and PSE that are located to maintain the promoter activity, a IoxP sequence therebetween, and another IoxP sequence either upstream of DSE or downstream of PSE; and
(c) two IoxP sequences are located so as to interpose the DNAs encoding an antisense RNA and a sense RNA.

8. The siRNA expression system according to claim 1, wherein said system comprises IoxP sequences in the form of any one of the following (a) to (d) so that the expression can be controlled:
(a) the promoter comprises DSE and PSE with a space therebetween, and in the space two IoxP sequences, one in the vicinity of DSE and the other is the vicinity of PSE;
(b) the promoter comprises DSE and PSE that are located to maintain the promoter activity, a IoxP sequence therebetween, and another IoxP upstream of DSE or downstream of PSE;
(c) two IoxPs are located so as to interpose the DNAs encoding an antisense RNA and a sense RNA; and
(d) two IoxPs are arranged so as to interpose a linker comprising a stop sequence.

9. The siRNA expression system according to any one of claims 1-8, wherein said vector is a plasmid vector.

10. The siRNA expression system according to any one of claims 1-8, wherein said vector is a viral vector.

11. The siRNA expression system according to any one of claim 1-8, wherein said vector is a dumbbell-shaped DNA vector.

12. A transformed cell comprising the siRNA expression system according to any one of claims 1 to 11.

13. The cell according to claim 12, wherein said cell is a mammalian cell.

14. A composition comprising the siRNA expression system according to any one of claims 1 to 11.

15. The composition according to claim 14, wherein said composition is a pharmaceutical composition.

16. A method for producing in vitro a cell in which the expression of a target gene mRNA is silenced, wherein said method comprises the steps of: introducing the siRNA expression system according to any one of claims 1 to 11 into cells, and selecting cells in which said siRNA expression system is introduced.

17. An intracellular siRNA library expression system which produces plural kinds of the siRNA according to any one of claims 1 to 11 in a mammalian cell.

18. The siRNA library expression system according claim 17, wherein siRNAs expressed by the system are composed of random RNA strands.

19. The siRNA library expression system according to claim 17, wherein said system is an assembly of multiple siRNA expression vectors that each targets a gene sequence comprising a coding region and/or a non-coding region.

20. The siRNA library expression system according to claim 17, wherein siRNAs expressed by the system are composed of RNA strands encoded by DNA fragments of any cDNA or genomic DNA, said fragment has the length of the siRNA to be expressed.

21. An assembly comprising plural kinds of the siRNA library expression systems according to any one of claims 17 to 20, wherein different siRNAs are expressed by each system in said assembly.

22. A method of searching for a functional gene, the method comprising the steps of:
(a) introducing the siRNA library expression system according to any one of claims 17 to 20 or the assembly of plural kinds of the siRNA library expression systems according to claim 21 into cells;
(b) selecting cells into which said siRNA library expression system or said assembly has been introduced; and
(c) analyzing the phenotype of the cells thus selected.

23. The method of searching' for a functional gene according to claim 22, wherein said method further comprising a step of screening for a functional gene based on the sequence of DNA coding for siRNA in the cell whose phenotype has been found altered as the result of the phenotype analysis.

24. A method for selecting a highly active siRNA, the method comprising the steps of:
(a) introducing the siRNA library expression system according to any one of claims 17 to 20, or the assembly of plural kinds of the siRNA library expression systems according to claim 21 into cells, and
(b) measuring the expression level of a specific gene or protein in the cells into which said siRNA library expression system or said assembly is introduced.

## Patentansprüche

1. Intrazelluläres siRNA-Expressionssystem, wobei die siRNA umfasst: Eine doppelsträngige Region, in der ein Antisinn-RNA-Strang und ein Sinn-RNA-Strang, der komplementär zu der Antisinn-RNA ist, ein Paar bilden, und gegen eine Region der Zielgen mRNA in der Säugerzelle gerichtet ist, und wobei die Antisinn- und Sinn-RNA-Stränge von einem Vektor produziert werden, der eine DNA umfasst, welche den Antisinn-RNA-Strang kodiert, und eine DNA umfasst, welche den Sinn-RNA-Strang kodiert, und einen oder mehrere Pol III-Promotoren, die in der Lage sind, die Antisinn- und Sinn-RNA-Stränge zu produzieren, wobei der Vektor ein beliebiges der folgenden (a) bis (c) umfasst:
a) eine Einheit, die eine Stamm-Schleifen-siRNA produziert, umfassend: Eine DNA, die den Antisinn-RNA-Strang kodiert und eine DNA, die den Sinn-RNA-Strang kodiert, der zu dem Antisinn-RNA-Strang komplementär ist, welche über einen Linker in der entgegengesetzten Richtung miteinander verknüpft sind, und einen Pol III-Promotor, der in der Lage ist, die Stamm-Schleifen-siRNA an beiden Seiten der Einheit zu produzieren, wobei die doppelsträngige RNA-Region der siRNA 1 bis 7 Fehlpaarungen und/oder 1 bis 7 Wölbungen enthält, und wobei eines der Nukleotide in der Fehlpaarung ein Guanin und das andere ein Uracil ist,
b) eine doppelsträngige DNA-Region, in der eine DNA, die den Antisinn-RNA-Strang kodiert und eine DNA, die den Sinn-Strang kodiert, der zu dem Antisinn-RNA-Strang komplementär ist, ein Paar bilden, und zwei einander zugewandte Pol III-Promotoren mit der doppelsträngigen DNA-Region dazwischen, die in der Lage sind, die Antisinn- und die Sinn-RNA-Stränge zu produzieren, oder
c) eine DNA, die den Antisinn-RNA-Strang kodiert, eine DNA, die den Sinn-RNA-Strang kodiert, der zu dem Antisinn-Strang komplementär ist, und Pol III-Promotoren an den 5'-Enden der Antisinn- und Sinn-kodierenden DNAs, die in der Lage sind, die Antisinn- und Sinn-RNA-Stränge zu produzieren.

2. Das siRNA-Expressionssystem nach Anspruch 1, wobei die doppelsträngige Region der siRNA, das durch das System exprimiert wird, 15 bis 49 Basenpaare lang ist.

3. Das siRNA-Expressionssystem nach Anspruch 1, wobei die doppelsträngige Region der siRNA, das durch das System exprimiert wird, 15 bis 35 Basenpaare lang ist.

4. Das siRNA-Expressionssystem nach Anspruch 1, wobei die doppelsträngige Region der siRNA, das durch das System exprimiert wird, 15 bis 30 Basenpaare lang ist.

5. Das siRNA-Expressionssystem nach einem der Ansprüche 1 bis 4, wobei der Pol III-Promotor der U6-Promotor ist.

6. Das siRNA-Expressionssystem nach einem der Ansprüche 1 bis 5, wobei der Promotor ein induzierbarer Promotor ist.

7. Das siRNA-Expressionssystem nach einem der Ansprüche 1 bis 6, wobei das System umfasst: LoxP-Sequenzen in der Form gemäß einem beliebigen der folgenden (a) bis (c), so dass die Expression kontrolliert werden kann:
(a) Der Promotor umfasst ein distales Sequenzelement (DSE) und ein proximales Sequenzelement (PSE) mit einem Abstand dazwischen, und in dem Abstand zwei LoxP-Sequenzen, eine in der Nachbarschaft von DSE und eine andere in der Nachbarschaft von PSE;
(b) der Promotor umfasst DSE und PSE, die so positioniert sind, dass sie die Promotoraktivität aufrechterhalten, eine LoxP-Sequenz dazwischen, und eine andere LoxP-Sequenz entweder stromaufwärts von DSE oder stromabwärts von PSE; und
(c) zwei LoxP-Sequenzen, die so positioniert sind, dass sie eingeschoben sind zwischen den DNAs, die eine Antisinn-RNA und eine Sinn-RNA kodieren.

8. Das siRNA-Expressionssystem nach Anspruch 1, wobei das System zwei LoxP-Sequenzen in der Form gemäß einem beliebigen der folgenden (a) bis (d) umfasst, so dass die Expression kontrolliert werden kann:
(a) Der Promotor umfasst DSE und PSE mit einem Abstand dazwischen und in dem Abstand zwei LoxP-Sequenzen, eine in der Nachbarschaft von DSE und die andere in der Nachbarschaft von PSE;
(b) der Promotor umfasst DSE und PSE, die so positioniert sind, dass die Promotoraktivität aufrechterhalten wird, eine LoxP-Sequenz dazwischen und eine andere LoxP stromaufwärts von DSE oder stromabwärts von PSE;
(c) zwei LoxP-Sequenzen, die so positioniert sind, dass sie eingeschoben sind zwischen den DNAs, die eine Antisinn-RNA oder eine Sinn-RNA kodieren; und
(d) zwei LoxPs, die so angeordnet sind, dass sie eingeschoben sind zwischen einem Linker, der eine Stoppsequenz umfasst.

9. Das siRNA-Expressionssystem nach einem der Ansprüche 1 - 8, wobei der Vektor ein Plasmidvektor ist.

10. Das siRNA-Expressionssystem nach einem der Ansprüche 1 - 8, wobei der Vektor ein viraler Vektor ist.

11. Das siRNA-Expressionssystem nach einem der Ansprüche 1 - 8, wobei der Vektor ein hantelförmiger DNA-Vektor ist.

12. Transformierte Zelle, umfassend das siRNA-Expressionssystem nach einem der Ansprüche 1 - 11.

13. Die Zelle nach Anspruch 12, wobei die Zelle eine Säugerzelle ist.

14. Zusammensetzung, umfassend das siRNA-Expressionssystem nach einem der Ansprüche 1 - 11.

15. Die Zusammensetzung nach Anspruch 14, wobei die Zusammensetzung ein Arzneimittel ist.

16. Verfahren zur *in vitro*-Herstellung einer Zelle, in der die Expression einer Zielgen-mRNA abgeschwächt ist, wobei das Verfahren die Schritte umfasst: Einfügen des siRNA-Expressionssystems nach einem der Ansprüche 1-11 in Zellen, und Selektieren der Zellen, in denen das siRNA-Expressionssystem eingefügt wurde.

17. Intrazelluläres siRNA-Genbank-Expressionssystem, welches eine Vielzahl von siRNAs nach einem der Ansprüche 1-11 einer in Säugerzelle produziert.

18. Das siRNA-Genbank-Expressionssystem nach Anspruch 17, wobei die siRNAs, die durch das System exprimiert werden, zusammengesetzt sind aus zufälligen RNA-Strängen.

19. Das siRNA-Genbank-Expressionssystem nach Anspruch 17, wobei das System eine Anordnung von einer Vielzahl von siRNA-Expressionsvektoren ist, die jeweils eine Gensequenz ansteuern, umfassend eine kodierende Region und/oder eine nicht kodierende Region.

20. Das siRNA-Genbank-Expressionssystem nach Anspruch 17, wobei die siRNAs, die durch das System exprimiert werden, zusammengesetzt sind aus RNA-Strängen, die durch die DNA-Fragmente einer beliebigen cDNA oder genomischen DNA kodiert werden, wobei das Fragment die Länge der siRNA hat, die exprimiert werden soll.

21. Anordnung, die eine Vielzahl von Arten von siRNA-Genbank-Expressionssystemen nach einem der Ansprüche 17 bis 20 umfasst, wobei unterschiedliche siRNAs durch jedes System in der Anordnung exprimiert werden.

22. Verfahren zum Durchsuchen nach einem funktionellen Gen, wobei das Verfahren die Schritte umfasst:
(a) Einfügen des siRNA-Genbank-Expressionssystems nach einem der Ansprüche 17 bis 20 oder der Anordnung von einer Vielzahl von Arten von siRNA-Genbank-Expressionssystemen nach Anspruch 21 in Zellen;
(b) Selektieren der Zellen, in denen das siRNA-Genbank-Expressionssystem oder die Anordnung eingefügt wurde; und
(c) Analysieren des Phänotyps der Zellen, die so selektiert wurden.

23. Das Verfahren zum Durchsuchen nach einem funktionellen Gen nach Anspruch 22, wobei das Verfahren weiterhin die Schritte umfasst: Das Screening nach funktionellen Genen, basierend auf der Sequenz der DNA, die die siRNA in der Zelle kodiert, deren Phänotyp als verändert gefunden wurde, als ein Ergebnis der Phänotypanalyse.

24. Verfahren zum Selektieren von hochaktiven siRNAs, wobei das Verfahren die Schritte umfasst:
(a) Einfügen des siRNA-Genbank-Expressionssystems nach einem der Ansprüche 17 bis 20, oder der Anordnung der Vielzahl von Arten von siRNA-Genbank-Expressionssystemen gemäß Anspruch 21 in Zellen, und
(b) Messen des Expressionsniveaus eines spezifischen Gens oder Proteins in den Zellen, in denen die siRNA-Genbank-Expressionssysteme oder die Anordnung eingefügt wurde.

## Revendications

1. Système d'expression d'ARNsi intracellulaire,
dans lequel ledit ARNsi comprend une région double brin, dans laquelle un brin d'ARN anti-sens et un brin d'ARN sens complémentaire à l'ARN anti-sens sont appariés, et sont dirigés contre une région d'un ARNm de gène cible dans une cellule mammifère, et
dans lequel lesdits brins d'ARN anti-sens et sens sont produits à partir d'un vecteur comprenant un ADN codant ledit brin d'ARN anti-sens et un ADN codant ledit brin d'ARN sens, et un ou plusieurs promoteurs pol III capables de produire lesdits brins d'ARN anti-sens et sens,
dans lequel ledit vecteur comprend n'importe quel élément parmi les points (a) à (c) suivants :
a) une unité de production d'ARNsi tige-boucle comprenant un ADN codant le brin d'ARN anti-sens et un ADN codant le brin d'ARN sens complémentaire du brin d'ARN anti-sens qui sont liés dans la direction opposée via un lieur, et un promoteur pol III capable de produire ledit ARNsi tige-boucle de chaque côté de ladite unité, dans lequel la région d'ARN double brin de l'ARNsi contient 1 à 7 mésappariements et/ou 1 à 7 saillies, et dans lequel un des nucléotides dans le mésappariement est la guanine, et l'autre est l'uracile ;
b) une région d'ADN double brin dans laquelle un ADN codant le brin d'ARN anti-sens et un ADN codant le brin d'ARN sens complémentaire du brin d'ARN anti-sens sont appariés, et deux promoteurs pol III se faisant face avec ladite région d'ADN double-brin entre eux qui sont capables de produire lesdits brins d'ARN anti-sens et sens, ou
c) un ADN codant le brin d'ARN anti-sens, un ADN codant le brin d'ARN sens complémentaire du brin d'ARN anti-sens, et des promoteurs pol III aux extrémités 5' des ADN de code anti-sens et sens qui sont capables de produire lesdits brins d'ARN anti-sens et sens.

2. Système d'expression d'ARNsi selon la revendication 1, dans lequel la région double brin de l'ARNsi exprimée par le système est de 15 à 49 pb de long.

3. Système d'expression d'ARNsi selon la revendication 1, dans lequel la région double brin de l'ARNsi exprimée par le système est de 15 à 35 pb de long.

4. Système d'expression d'ARNsi selon la revendication 1, dans lequel la région double brin de l'ARNsi exprimée par le système est de 15 à 30 pb de long.

5. Système d'expression d'ARNsi selon l'une quelconque des revendications 1 à 4, dans lequel ledit promoteur pol III est un promoteur U6.

6. Système d'expression d'ARNsi selon l'une quelconque des revendications 1 à 5, dans lequel ledit promoteur est un promoteur inductible.

7. Système d'expression d'ARNsi selon l'une quelconque des revendications 1 à 6, dans lequel ledit système comprend des séquences IoxP sous la forme de l'un quelconque des éléments (a) à (c), de sorte que l'expression puisse être contrôlée :
(a) le promoteur comprend un élément de séquence distale (DSE) et un élément de séquence proximale (PSE) avec un espace entre eux, et dans l'espace, deux séquences IoxP, une à proximité du DSE et l'autre à proximité du PSE ;
(b) le promoteur comprend un DSE et un PSE qui sont situés de façon à maintenir l'activité du promoteur, une séquence IoxP entre eux, et une autre séquence IoxP soit en amont du DSE ou en aval du PSE ; et
(c) deux séquences IoxP sont situées de façon à intercaler les ADNs codant un ARN anti-sens et un ARN sens.

8. Système d'expression d'ARNsi selon la revendication 1, dans lequel ledit système comprend des séquences IoxP, sous la forme de l'un quelconque des éléments (a) à (d), de sorte que l'expression puisse être contrôlée :
(a) le promoteur comprend un DSE et un PSE avec un espace entre eux, et dans l'espace, deux séquences IoxP, une à proximité du DSE et l'autre à proximité du PSE ;
(b) le promoteur comprend un DSE et un PSE qui sont situés de façon à maintenir l'activité du promoteur, une séquence IoxP entre eux, et un autre IoxP en amont du DSE ou en aval du PSE ;
(c) deux IoxP sont situés de façon à intercaler les ADNs codant un ARN anti-sens et un ARN sens ; et
(d) deux IoxP sont disposés de façon à interposer un bras de liaison comprenant une séquence d'arrêt.

9. Système d'expression d'ARNsi selon l'une quelconque des revendications 1 à 8, dans lequel ledit vecteur est un vecteur plasmide.

10. Système d'expression d'ARNsi selon l'une quelconque des revendications 1 à 8, dans lequel ledit vecteur est un vecteur viral.

11. Système d'expression d'ARNsi selon l'une quelconque des revendications 1 à 8, dans lequel ledit vecteur est un vecteur d'ADN en forme d'haltère.

12. Cellule transformée comprenant le système d'expression d'ARNsi, selon l'une quelconque des revendications 1 à 11.

13. Cellule selon la revendication 12, ladite cellule étant une cellule mammifère.

14. Composition comprenant le système d'expression d'ARNsi selon l'une quelconque des revendications 1 à 11.

15. Composition selon la revendication 14, ladite composition étant une composition pharmaceutique.

16. Procédé de production in vitro d'une cellule, dans laquelle l'expression d'un ARNm génique cible est tue, ledit procédé comprenant les étapes consistant à : introduire le système d'expression d'ARNsi, selon l'une quelconque des revendications 1 à 11 dans des cellules, et choisir les cellules dans lesquelles ledit système d'expression d'ARNsi est introduit.

17. Système d'expression d'une banque d'ARNsi intracellulaire qui produit plusieurs types de l'ARNsi, selon l'une quelconque des revendications 1 à 11 dans une cellule mammifère.

18. Système d'expression de banque d'ARNsi selon la revendication 17, dans lequel les ARNsi exprimés par le système sont composés de brins d'ARN aléatoires.

19. Système d'expression de banque d'ARNsi selon la revendication 17, dans lequel ledit système est un assemblage de multiples vecteurs d'expression d'ARNsi qui ciblent chacun une séquence génique comprenant une région codante et/ou une région non-codante.

20. Système d'expression de banque d'ARNsi selon la revendication 17, dans lequel les ARNsi exprimés par le système sont composés de brins d'ARN codés par des fragments d'ADN à base d'ADNc ou d'ADN génomique, ledit fragment ayant la longueur de l'ARNsi à exprimer.

21. Assemblage comprenant plusieurs types de systèmes d'expression de banque d'ARNsi, selon l'une quelconque des revendications 17 à 20, dans lequel différents ARNsi sont exprimés par chaque système dans ledit assemblage.

22. Procédé de recherche d'un gène fonctionnel, le procédé comprenant les étapes consistant à :
(a) introduire le système d'expression de banque d'ARNsi, selon l'une quelconque des revendications 17 à 20 ou l'assemblage de plusieurs types de systèmes d'expression de la banque d'ARNsi selon la revendication 21 dans les cellules ;
(b) choisir des cellules, dans lesquelles ledit système d'expression de banque d'ARNsi ou ledit assemblage a été introduit ; et
(c) analyser le phénotype des cellules ainsi choisies.

23. Procédé de recherche d'un gène fonctionnel selon la revendication 22, ledit procédé comprenant en outre une étape de criblage pour un gène fonctionnel basé sur la séquence de codage d'ADN pour l'ARNsi dans la cellule dont le phénotype s'est avéré altéré du fait de l'analyse de phénotype.

24. Procédé de sélection d'un ARNsi hautement actif, le procédé comprenant les étapes consistant à :
(a) introduire le système d'expression de banque d'ARNsi, selon l'une quelconque des revendications 17 à 20, ou l'assemblage de plusieurs types des systèmes d'expression de banque d'ARNsi selon la revendication 21 dans les cellules, et
(b) mesurer le niveau d'expression d'un gène spécifique ou de la protéine dans les cellules, dans lesquelles ledit système d'expression de banque d'ARNsi ou ledit assemblage est introduit.


### SEQUENCE LISTING

<110> CENTER FOR ADVANCED SCIENCE AND TECHNOLOGY INCUBATION, LTD.
<120> siRNA expression systems and methods for generating cells with disrupting gene functions using the systems.
<130> SEN-A0124P2
<140>
   <141>
<150> JP 2001-363385
   <151> 2001-11-28
<150> PCT/JP02/11293
   <151> 2002-10-30
<160> 57
<170> PatentIn Ver. 2.1
<210> 1
   <211> 19
   <212> DNA
   <213> Artificial Sequence
<220>
   <223> Description of Artificial Sequence:Artificially Synthesized Sequence
<400> 1
   gctatgaaac gatatgggc 19
<210> 2
   <211> 19
   <212> DNA
   <213> Artificial Sequence
<220>
   <223> Description of Artificial Sequence:Artificially Synthesized Sequence
<400> 2
   gcccatatcg tttcatagc 19
<210> 3
   <211> 20
   <212> DNA
   <213> Artificial Sequence
<220>
   <223> Description of Artificial Sequence:Artificially Synthesized Sequence
<400> 3
   gttcgtcaca tctcatctac 20
<210> 4
   <211> 19
   <212> DNA
   <213> Artificial Sequence
<220>
   <223> Description of Artificial Sequence:Artificially Synthesized Sequence
<400> 4
   gtagatgaga tgtgacgaa 19
<210> 5
   <211> 19
   <212> DNA
   <213> Artificial Sequence
<220>
   <223> Description of Artificial Sequence:Artificially Synthesized Sequence
<400> 5
   gtgcgctgct ggtgccaac 19
<210> 6
   <211> 19
   <212> DNA
   <213> Artificial Sequence
<220>
   <223> Description of Artificial Sequence:Artificially Synthesized Sequence
<400> 6
   gttggcacca gcagcgcac 19
<210> 7
   <211> 19
   <212> DNA
   <213> Artificial Sequence
<220>
   <223> Description of Artificial Sequence:Artificially Synthesized Sequence
<400> 7
   atgtacacgt tcgtcacat 19
<210> 8
   <211> 19
   <212> DNA
   <213> Artificial Sequence
<220>
   <223> Description of Artificial Sequence:Artificially Synthesized Sequence
<400> 8
   atgtgacgaa cgtgtacat 19
<210> 9
   <211> 19
   <212> DNA
   <213> Artificial Sequence
<220>
   <223> Description of Artificial Sequence:Artificially Synthesized Sequence
<400> 9
   gtagcgcggt gtattatac 19
<210> 10
   <211> 19
   <212> DNA
   <213> Artificial Sequence
<220>
   <223> Description of Artificial Sequence:Artificially Synthesized Sequence
<400> 10
   gtataataca ccgcgctac 19
<210> 11
   <211> 21
   <212> RNA
   <213> Artificial Sequence
<220>
   <223> Description of Artificial Sequence:Artificially Synthesized Sequence
<400> 11
   gcuaugaaac gauaugggcu u 21
<210> 12
   <211> 21
   <212> RNA
   <213> Artificial Sequence
<220>
   <223> Description of Artificial Sequence:Artificially Synthesized Sequence
<400> 12
   gcccauaucg uuucauagcu u 21
<210> 13
   <211> 22
   <212> RNA
   <213> Artificial Sequence
<220>
   <223> Description of Artificial Sequence:Artificially Synthesized Sequence
<400> 13
   guucgucaca ucucaucuac uu 22
<210> 14
   <211> 21
   <212> RNA
   <213> Artificial Sequence
<220>
   <223> Description of Artificial Sequence:Artificially Synthesized Sequence
<400> 14
   guagaugaga ugugacgaau u 21
<210> 15
   <211> 21
   <212> RNA
   <213> Artificial Sequence
<220>
   <223> Description of Artificial Sequence:Artificially Synthesized Sequence
<400> 15
   gugcgcugcu ggugccaacu u 21
<210> 16
   <211> 21
   <212> RNA
   <213> Artificial Sequence
<220>
   <223> Description of Artificial Sequence:Artificially Synthesized Sequence
<400> 16
   guuggcacca gcagcgcacu u 21
<210> 17
   <211> 21
   <212> RNA
   <213> Artificial Sequence
<220>
   <223> Description of Artificial Sequence:Artificially Synthesized Sequence
<400> 17
   auguacacgu ucgucacauu u 21
<210> 18
   <211> 21
   <212> RNA
   <213> Artificial Sequence
<220>
   <223> Description of Artificial Sequence:Artificially Synthesized Sequence
<400> 18
   augugacgaa cguguacauu u 21
<210> 19
   <211> 21
   <212> RNA
   <213> Artificial Sequence
<220>
   <223> Description of Artificial Sequence:Artificially Synthesized Sequence
<400> 19
   guagcgcggu guauuauacu u 21
<210> 20
   <211> 21
   <212> RNA
   <213> Artificial Sequence
<220>
   <223> Description of Artificial Sequence:Artificially Synthesized Sequence
<400> 20
   guauaauaca ccgcgcuacu u 21
<210> 21
   <211> 49
   <212> DNA
   <213> Artificial Sequence
<220>
   <223> Description of Artificial Sequence:Artificially Synthesized Sequence
<400> 21
   gtgcgctgct ggtgccaacg ugugcugucc gttggcacca gcagcgcac 49
<210> 22
   <211> 53
   <212> RNA
   <213> Artificial Sequence
<220>
   <223> Description of Artificial Sequence:Artificially Synthesized Sequence
<400> 22
   gugcgcugcu ggugccaacc cgugugcugu ccggguuggc accagcagcg cac 53
<210> 23
   <211> 53
   <212> RNA
   <213> Artificial Sequence
<220>
   <223> Description of Artificial Sequence:Artificially Synthesized Sequence
<400> 23
   gugcgcuguu ggugucaacc cgugugcugu ccggguuggc accagcagcg cac 53
<210> 24
   <211> 54
   <212> RNA
   <213> Artificial Sequence
<220>
   <223> Description of Artificial Sequence:Artificially Synthesized Sequence
<400> 24
   gugcgcugcu ggugcucaac ccgugugcug uccggguugg caccagcagc gcac 54
<210> 25
   <211> 19
   <212> DNA
   <213> Artificial Sequence
<220>
   <223> Description of Artificial Sequence:Artificially Synthesized Sequence
<220>
   <221> misc_feature
   <222> (1) .. (19)
   <223> "n" = any one base of a, t, g, or c.
<400> 25
   nnnnnnnnnn nnnnnnnnn 19
<210> 26
   <211> 81
   <212> DNA
   <213> Artificial Sequence
<220>
   <223> Description of Artificial Sequence:Artificially Synthesized Sequence
<400> 26
   ttcggcaggt ccggtcgacc ctgcacgcgg ccaaggccga aaaggccgcg gccgcaagca 60
   ggctcgaccg gacctgccga a 81
<210> 27
   <211> 119
   <212> DNA
   <213> Artificial Sequence
<220>
   <223> Description of Artificial Sequence:Artificially Synthesized Sequence
<220>
   <221> misc_feature
   <222> (1) .. (19)
   <223> "n" = any one base of a, t, g, or c.
<220>
   <221> misc_feature
   <222> (101).. (119)
   <223> "n" = any one base of a, t, g, or c.
<400> 27
   nnnnnnnnnn nnnnnnnnnt tcggcaggtc cggtcgaccc tgcacgcggc caaggccgaa 60
   aaggccgcgg ccgcaagcag gctcgaccgg acctgccgaa nnnnnnnnnn nnnnnnnnn 119
<210> 28
   <211> 39
   <212> DNA
   <213> Artificial Sequence
<220>
   <223> Description of Artificial Sequence:Artificially Synthesized Sequence
<400> 28
   ggctcgagaa gcttggcgcg ccgctcttcg cgccaaaaa 39
<210> 29
   <211> 37
   <212> DNA
   <213> Artificial Sequence
<220>
   <223> Description of Artificial Sequence:Artificially Synthesized Sequence
<400> 29
   tttttggcgc gaagagcggc gcgccaagct tctcgag 37
<210> 30
   <211> 195
   <212> DNA
   <213> Artificial Sequence
<220>
   <223> Description of Artificial Sequence:Artificially Synthesized Sequence
<220>
   <221> misc_feature
   <222> (39) .. (58)
   <223> "n" = any one base of a, t, g, or c.
<220>
   <221> misc_feature
   <222> (139).. (158)
   <223> "n" = any one base of a, t, g, or c.
<400> 30
   ggctcgagaa gcttggcgcg ccgctcttcg cgccaaaaan nnnnnnnnnn nnnnnnnntt 60
   cggcaggtcc ggtcgaccct gcacgcggcc aaggccgaaa aggccgcggc cgcaagcagg 120
   ctcgaccgga cctgccgaan nnnnnnnnnn nnnnnnnntt tttggcgcga agagcggcgc 180
   gccaagcttc tcgag 195
<210> 31
   <211> 152
   <212> DNA
   <213> Artificial Sequence
<220>
   <223> Description of Artificial Sequence:Artificially Synthesized Sequence
<220>
   <221> misc_feature
   <222> (39) .. (55)
   <223> "n" = any one base of a, t, g, or c.
<220>
   <221> misc_feature
   <222> (137) .. (152)
   <223> "n" = any one base of a, t, g, or c.
<400> 31
   ggctcgagaa gcttggcgcg ccgctcttcg cgccaaaaan nnnnnnnnnn nnnnnttcgg 60
   caggtccggt cgaccctgct tgcggccgcg gccttttcgg ccttggccgc gtgcagggtc 120
   gaccggacct gccgaannnn nnnnnnnnnn nn 152
<211> 152
   <212> DNA
   <213> Artificial Sequence
<220>
   <223> Description of Artificial Sequence:Artificially Synthesized Sequence
<220>
   <221> misc_feature
   <222> (1) .. (16)
   <223> "n" = any one base of a, t, g, or c.
<220>
   <221> misc_feature
   <222> (98).. (113)
   <223> "n" = any one base of a, t, g, or c.
<400> 32
<210> 33
   <211> 142
   <212> DNA
   <213> Artificial Sequence
<220>
   <223> Description of Artificial Sequence:Artificially Synthesized Sequence
<220>
   <221> misc_feature
   <222> (24) .. (42)
   <223> "n" = any one base of a, t, g, or c.
<220>
   <221> misc_feature
   <222> (124)..(142)
   <223> "n" = any one base of a, t, g, or c.
<400> 33
   cgcgccgctc ttcgcgccaa aaannnnnnn nnnnnnnnnn nnttcggcag gtccggtcga 60
   ccctgcttgc ggccgcggcc ttttcggcct tggccgcgtg cagggtcgac cggacctgcc 120
   gaannnnnnn nnnnnnnnnn nn 142
<210> 34
   <211> 138
   <212> DNA
   <213> Artificial Sequence
<220>
   <223> Description of Artificial Sequence:Artificially Synthesized Sequence
<220>
   <221> misc_feature
   <222> (1) .. (19)
   <223> "n" = any one base of a, t, g, or c.
<220>
   <221> misc_feature
   <222> (101).. (119)
   <223> "n" = any one base of a, t, g, or c.
<400> 34
   nnnnnnnnnn nnnnnnnnnt tcggcaggtc cggtcgaccc tgcacgcggc caaggccgaa 60
   aaggccgcgg ccgcaagcag ggtcgaccgg acctgccgaa nnnnnnnnnn nnnnnnnnnt 120
   ttttggcgcg aagagcgg 138
<210> 35
   <211> 43
   <212> DNA
   <213> Artificial Sequence
<220>
   <223> Description of Artificial Sequence:Artificially Synthesized Sequence
<220>
   <221> misc_feature
   <222> (6).. (43)
   <223> "n" = any one base of a, t, g, or c.
<400> 35
   aaaaannnnn nnnnnnnnnn nnnnnnnnnn nnnnnnnnnn nnn 43
<210> 36
   <211> 43
   <212> DNA
   <213> Artificial Sequence
<220>
   <223> Description of Artificial Sequence:Artificially Synthesized Sequence
<220>
   <221> misc_feature
   <222> (1).. (38)
   <223> "n" = any one base of a, t, g, or c.
<400> 36
   nnnnnnnnnn nnnnnnnnnn nnnnnnnnnn nnnnnnnntt ttt 43
<210> 37
   <211> 47
   <212> DNA
   <213> Artificial Sequence
<220>
   <223> Description of Artificial Sequence:Artificially Synthesized Sequence
<220>
   <221> misc_feature
   <222> (6).. (24)
   <223> "n" = any one base of a, t, g, or c.
<220>
   <221> misc_feature
   <222> (29).. (47)
   <223> "n" = any one base of a, t, g, or c.
<400> 37
   aaaaannnnn nnnnnnnnnn nnnncgaann nnnnnnnnnn nnnnnnn 47
<210> 38
   <211> 47
   <212> DNA
   <213> Artificial Sequence
<220>
   <223> Description of Artificial Sequence:Artificially Synthesized Sequence
<220>
   <221> misc_feature
   <222> (1).. (19)
   <223> "n" = any one base of a, t, g, or c.
<220>
   <221> misc_feature
   <222> (24) .. (42)
   <223> "n" = any one base of a, t, g, or c.
<400> 38
   nnnnnnnnnn nnnnnnnnnt tcgnnnnnnn nnnnnnnnnn nnttttt 47
<210> 39
   <211> 24
   <212> DNA
   <213> Artificial Sequence
<220>
   <223> Description of Artificial Sequence:Artificially Synthesized Sequence
<400> 39
   cccgtgccct ggcccaccct cgtg 24
<210> 40
   <211> 24
   <212> DNA
   <213> Artificial Sequence
<220>
   <223> Description of Artificial Sequence:Artificially Synthesized Sequence
<400> 40
   cacgagggtg ggccagggca cggg 24
<210> 41
   <211> 24
   <212> DNA
   <213> Artificial Sequence
<220>
   <223> Description of Artificial Sequence:Artificially Synthesized Sequence
<400> 41
   accaggatgg gcaccacccc ggtg 24
<210> 42
   <211> 24
   <212> DNA
   <213> Artificial Sequence
<220>
   <223> Description of Artificial Sequence:Artificially Synthesized Sequence
<400> 42
   caccggggtg gtgcccatcc tggt 24
<210> 43
   <211> 19
   <212> DNA
   <213> Artificial Sequence
<220>
   <223> Description of Artificial Sequence:Artificially Synthesized Sequence
<400> 43
   gtgcgctgct ggtgccaac 19
<210> 44
   <211> 21
   <212> DNA
   <213> Artificial Sequence
<220>
   <223> Description of Artificial Sequence:Artificially Synthesized Sequence
<400> 44
   gtgcgctgct ggtgccaacc c 21
<210> 45
   <211> 21
   <212> DNA
   <213> Artificial Sequence
<220>
   <223> Description of Artificial Sequence:Artificially Synthesized Sequence
<400> 45
   gtgcgttgtt ggtgttaatc c 21
<210> 46
   <211> 21
   <212> DNA
   <213> Artificial Sequence
<220>
   <223> Description of Artificial Sequence:Artificially Synthesized Sequence
<400> 46
   gtgcgctgct ggtgtcaacc c 21
<210> 47
   <211> 21
   <212> DNA
   <213> Artificial Sequence
<220>
   <223> Description of Artificial Sequence:Artificially Synthesized Sequence
<400> 47
   gtgcggtggt ggtgggaagc c 21
<210> 48
   <211> 21
   <212> DNA
   <213> Artificial Sequence
<220>
   <223> Description of Artificial Sequence:Artificially Synthesized Sequence
<400> 48
   gtgcgttggt ggtggcaacc c 21
<210> 49
   <211> 21
   <212> DNA
   <213> Artificial Sequence
<220>
   <223> Description of Artificial Sequence:Artificially Synthesized Sequence
<400> 49
   gtgcgctcat ggtaccaacc c 21
<210> 50
   <211> 21
   <212> DNA
   <213> Artificial Sequence
<220>
   <223> Description of Artificial Sequence:Artificially Synthesized Sequence
<400> 50
   gtgcgctgct ggtgtcaacc c 21
<210> 51
   <211> 21
   <212> DNA
   <213> Artificial Sequence
<220>
   <223> Description of Artificial Sequence:Artificially Synthesized Sequence
<400> 51
   gtgcgctgtt ggtgtcaacc c 21
<210> 52
   <211> 21
   <212> DNA
   <213> Artificial Sequence
<220>
   <223> Description of Artificial Sequence:Artificially Synthesized Sequence
<400> 52
   gtgtgttgtt ggtgtcaatc c 21
<210> 53
   <211> 21
   <212> DNA
   <213> Artificial Sequence
<220>
   <223> Description of Artificial Sequence:Artificially Synthesized Sequence
<400> 53
   gtgtgttgtt ggtgttaatt c 21
<210> 54
   <211> 22
   <212> DNA
   <213> Artificial Sequence
<220>
   <223> Description of Artificial Sequence:Artificially Synthesized Sequence
<400> 54
   gtgcgttgtt ggtgtttaat cc 22
<210> 55
   <211> 23
   <212> DNA
   <213> Artificial Sequence
<220>
   <223> Description of Artificial Sequence:Artificially Synthesized Sequence
<400> 55
   gtgcgctgtc tggtgctcaa ccc 23
<210> 56
   <211> 25
   <212> DNA
   <213> Artificial Sequence
<220>
   <223> Description of Artificial Sequence:Artificially Synthesized Sequence
<400> 56
   gtgtcgctgt ctggtgctca actcc 25
<210> 57
   <211> 53
   <212> RNA
   <213> Artificial Sequence
<220>
   <223> Description of Artificial Sequence:Artificially Synthesized Sequence
<400> 57
   gugcguuguu gguguuaauc cgugugcugu ccggguuggc accagcagcg cac 53